(19) Europäisches Patentamt
European Patent Office
Office européen des brevets

(11) **EP 3 004 051 B1**

(12) **EUROPEAN PATENT SPECIFICATION**

(45) Date of publication and mention of the grant of the patent:
**14.11.2018 Bulletin 2018/46**

(21) Application number: **14730468.7**

(22) Date of filing: **28.05.2014**

(51) Int Cl.:
***C07C 315/02*** *(2006.01)*

(86) International application number:
**PCT/EP2014/061097**

(87) International publication number:
**WO 2014/191475 (04.12.2014 Gazette 2014/49)**

(54) **PROCESS FOR THE OXIDATION OF SULFOXIDES**

VERFAHREN ZUR OXIDATION VON SULFOXIDEN

PROCÉDÉ D'OXYDATION DE SULFOXYDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2013 EP 13169755**

(43) Date of publication of application:
**13.04.2016 Bulletin 2016/15**

(73) Proprietor: **BASF SE**
**67056 Ludwigshafen am Rhein (DE)**

(72) Inventors:
- **PARVULESCU, Andrei-Nicolae**
  **69117 Heidelberg (DE)**
- **MÜLLER, Ulrich**
  **67435 Neustadt (DE)**
- **SPIELMANN, Jan**
  **68199 Mannheim (DE)**
- **VOGEL, Wilfried**
  **67125 Dannstadt-Schauernheim (DE)**
- **GAO, Jun**
  **67434 Neustadt (DE)**

(74) Representative: **BASF IP Association**
**BASF SE**
**G-FLP-C006**
**67056 Ludwigshafen (DE)**

(56) References cited:
**CN-A- 102 838 516**
**US-A- 4 287 366**
**US-A1- 2010 120 869**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to a process for the oxidation of a sulfoxide, which process comprising reacting the sulfoxide with hydrogen peroxide in the presence of a catalyst comprising a porous titanium-containing silicate as a catalytically active material.

Sulfones which are chemical compounds with the general structural formula R1-S(=O)$_2$-R$_2$ where R$_1$ and R$_2$ are organic moieties are widely used in chemical industry. For example, diaryl-sulfones, such as 4,4'-dichlorodiphenylsulfone are important precursors for the production of polyarylene sulfones used, for example, as thermostable polymers.

RU-C-2158257 discloses a process for the preparation of 4,4'-dichlorodiphenyl sulfone comprising reacting, in a first step, thionyl chloride with chlorobenzene in the presence of aluminum chloride to obtain 4,4'-dichlorodiphenyl sulfoxide. In a second step, the sulfoxide is oxidized to 4,4'-dichlorodiphenyl sulfone making use of a mixture comprising hydrogen peroxide and acetic acid.

CN-A-102351757 similarly discloses the synthesis of 4,4'-dichlorodiphenyl sulfone which comprises reacting thionyl chloride with chlorobenzene in the presence of aluminum chloride to 4,4'-dichlorodiphenyl sulfoxide. In a second step, the sulfoxide is oxidized with hydrogen peroxide to 4,4'-dichlorodiphenyl sulfone in the presence of an organoselenic acid as catalyst.

CN-A-102351756 discloses a 4,4'-dichlorodiphenyl sulfone synthesis wherein thionyl chloride is reacted with chlorobenzene in the presence of aluminum trioxide to 4,4'-dichlorodiphenyl sulfoxide. Subsequently, 4,4'-dichlorodiphenyl sulfoxide is oxidized with hydrogen peroxide to 4,4'-dichlorodiphenyl sulfone using heteropolyacids such as phosphotungstic acid and silicotungstic acid supported on activated carbon as catalysts.

In WO-A-2012/143281 a one-pot synthesis for the preparation of a sulfone is disclosed wherein an acid selected from the group consisting of sulfuric acid, arene sulfonic acid and oleum is reacted with fluorinated anhydride and at least one halobenzene. For this reaction, catalysts are disclosed which can be homogeneous or heterogeneous. Among the heterogeneous catalysts, aluminosilicates are described. As aluminosilicates, an H-beta zeolite is described which has a silica : alumina ratio of not more than 40. With regard to the temperature profile, the reaction described in WO-A-2012/143281 is very complex since for individual steps of the reaction, three different temperatures T1, T2 and T3 have to be realized.

US-A-4287366 discloses a process for preparing bisphenol sulfone derivatives via oxidation with hydrogen peroxide, in the presence of alkali metal hydroxide in an amount equimolar relative to said compound in water and/or an organic solvent. US-A-2010/120869 relates to compounds and methods for PKC theta inhibition, wherein the synthesis of 4-[substituted (phenylsulfinyl and phenylsulfonyl)]-anilines from 4-(substituted phenylthio)-anilines by using hydrogen peroxide in the absence of a catalyst is disclosed.

CN-A-102838516 discloses a preparation method for sulfoxides and sulfones. As starting materials, thioethers are employed. According to this document, either a sulfoxide or a sulfone is prepared from the thioether using a titanium-containing zeolite as a catalyst, wherein microporous TS-1 and mesoporous Ti-MCM-41 are used. In particular, the document is silent on a process which makes use of a sulfoxide as starting material for the preparation of a sulfone with a catalyst comprising a Ti-MWW zeolite.

In most processes of the prior art, comparatively complex catalyst systems such as supported heteropoly acids or organoselenic acids and/or complex reaction sequences are taught.

Therefore, it was a subject of the present invention to provide an advantageous process for the preparation of a sulfone.

Surprisingly, it was found that such an advantageous process can be realized if a specific heterogeneous catalyst comprising titanium is employed and a sulfoxide is oxidized in the presence of this catalyst to obtain the sulfone.

Therefore, the present invention relates to a process for oxidizing a sulfoxide to the respective sulfone, said process comprising

(i) reacting the sulfoxide with hydrogen peroxide in the presence of a catalyst, obtaining a mixture (M) comprising the sulfone and the catalyst,

wherein the catalyst comprises a porous titanium-containing silicate as a catalytically active material and wherein the porous titanium-containing silicate comprised in the catalyst is a titanium-containing zeolitic material having a zeolitic framework structure comprising titanium and silicon, wherein the framework structure of the titanium-containing zeolitic material comprised in the catalyst is the MWW framework structure.

<u>Step (i)</u>

In step (i), a sulfoxide is reacted with hydrogen peroxide in the presence of a catalyst, thereby obtaining a mixture (M) comprising the sulfone and the catalyst, wherein the catalyst comprises a porous titanium-containing silicate

as a catalytically active material and wherein the porous titanium-containing silicate comprised in the catalyst is a titanium-containing zeolitic material having a zeolitic framework structure comprising titanium and silicon, wherein the framework structure of the titanium-containing zeolitic material comprised in the catalyst is the MWW framework structure.

Preferably, the sulfoxide used as educt in step (i) has a structure according to formula (I)

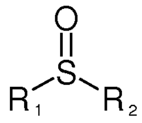

(I),

and the respective sulfone obtained as product has a structure according to formula (II)

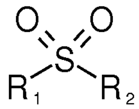

(II),

wherein $R_1$ and $R_2$ are independently from one another linear or branched, substituted or unsubstituted alkyl residues preferably having from 1 to 20 carbon atoms, linear or branched, substituted or unsubstituted alkenyl residues preferably having from 2 to 20 carbon atoms, or substituted or unsubstituted aryl or heteroaryl residues preferably having from 5 to 20 carbon atoms. Preferably, $R_1$ and $R_2$ are independently from one another substituted or unsubstituted aryl residues, more preferably substituted aryl residues. Preferably, the substituents are chosen from the group consisting of halogen such as F, Cl, Br, or I, hydroxyl, linear or branched alkyl residues preferably having from 1 to 10 carbon atoms, linear or branched alkyloxy residues preferably having from 1 to 10 carbon atoms, linear or branched alkenyl residues preferably having from 2 to 10 carbon atoms, aryl residues preferably having from 5 to 10 carbon atoms, heteroaryl residues preferably having from 5 to 10 carbon atoms, and combinations of two or more thereof. Preferably, the heteroatoms of the heteroaryl residues are chosen from the group consisting of N, P, O, and S. More preferably, $R_1$ and $R_2$ are independently from one another substituted aryl residues having from 5 to 10 carbon atoms, more preferably from 6 to 10 carbon atoms, wherein the substituents are preferably halogen, such as F, Cl, Br, or I, or hydroxyl, more preferably Cl or hydroxyl.

More preferably, the sulfoxide is 4,4'-dichlorodiphenyl sulfoxide according to formula (III). Accordingly, it is preferred that the sulfone obtained in step (i) is 4,4'-dichlorodiphenyl sulfone according to formula (IV):

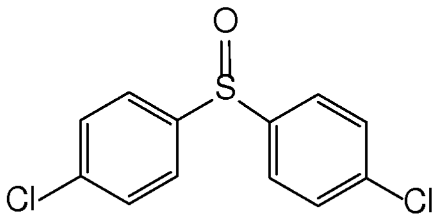

(III)

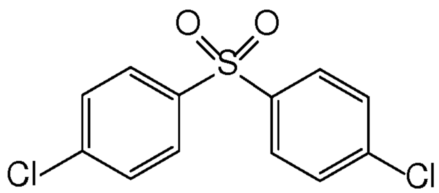

(IV)

Also preferably, the sulfoxide is 4,4'-dihydroxydiphenyl sulfoxide according to formula (IIIa). Accordingly, it is also preferred that the sulfone obtained in step (i) is 4,4'-didihydroxydiphenyl sulfone according to formula (IVa):

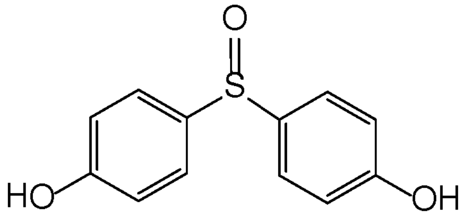

(IIIa)

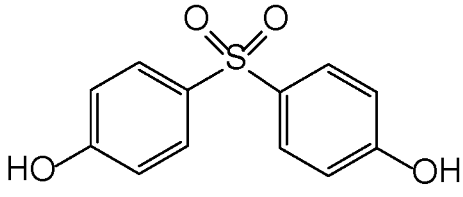

(IVa)

The catalyst used in the process of the present invention comprises a porous titanium-containing silicate as a catalytically active material, wherein the porous titanium-containing silicate comprised in the catalyst is a titanium-containing zeolitic material having a zeolitic framework structure comprising titanium and silicon, wherein the framework structure of the titanium-containing zeolitic material comprised in the catalyst is the MWW framework structure.

Depending on their pore size, porous silicates can have micropores, i.e. pores having a pore size of less than 2 nanometer, and/or mesopores, i.e. pores having a pore in the range of 2 to 50 nanometer, and/or macropores, i.e. pores having a pore size of more than 50 nanometer. Said pore sizes are to be understood as being determined according to the method as described in DIN 66135 and DIN 66134. Preferably, the porous titanium-containing silicate comprised in the catalyst, which is a titanium-containing zeolitic material having a zeolitic framework structure comprising titanium and silicon, wherein the framework structure of the titanium-containing zeolitic material comprised in the catalyst is the MWW framework structure of the present invention comprises micropores and/or mesopores. More preferably, the porous titanium-containing silicate comprised in the catalyst, which is a titanium-containing zeolitic material having a zeolitic framework structure comprising titanium and silicon, wherein the framework structure of the titanium-containing zeolitic material comprised in the catalyst is the MWW framework structure of the present invention comprises micropores.

The porous titanium-containing silicate comprised in the catalyst is a titanium-containing zeolitic material having a zeolitic framework structure comprising titanium and silicon, wherein the framework structure of the titanium-containing zeolitic material comprised in the catalyst is the MWW framework structure.

The zeolitic material which has a zeolitic MWW framework structure comprising titanium and silicon may be produced by substituting titanium into the tetrahedral position of the silicate framework, so that aluminum and/or silicon atoms are at least partly replaced. A zeolitic material having a zeolitic framework structure comprising titanium and silicon, which is preferably aluminum free, can be prepared according to all conceivable methods. In principle, a zeolitic framework structure comprising titanium and silicon can be prepared either by direct synthesis and/or secondary synthesis.

The titanium comprised in the zeolitic MWW framework structure can be incorporated in the framework structure according to all conceivable methods. For example, it is possible to synthesize the zeolitic material based on at least one suitable titanium source, at least one suitably silicon source, and optionally in the presence of at least one suitable template compound. Further, it is conceivable to prepare in a first step a zeolitic material containing a heteroatom other than titanium, such as, for example, aluminum and/or boron, suitably and at least partially remove the heteroatom other than titanium from the zeolitic framework, and introduce titanium in the zeolitic framework at least a portion of the framework sites previously having been occupied by the heteroatom other than titanium.

In addition to the titanium, the zeolitic MWW framework may include at least one further heteroatom. Conceivable further heteroatoms include, but are no restricted to, Al, Zr, V, Nb, Ta, Cr, Mo, W, Mn, Fe, Co, Ni, Zn, Ga, Ge, In, Sn, Pb. Preferably, the zeolitic framework of the present invention is essentially free of aluminum. The term "essentially free of aluminum" as used in this context of the present invention relates to a zeolitic framework which comprises 500 ppm or less aluminum, preferably, 300 ppm or less aluminum, more preferably 200 ppm or less aluminum based on the total weight of the zeolitic framework of the zeolitic material. Therefore, more preferred further heteroatoms are selected from the group consisting of Zr, V, Nb, Ta, Cr, Mo, W, Mn, Fe, Co, Ni, Zn, Ga, Ge, In, Sn, Pb, and combinations of two or more thereof.

Preferably, at least 50 weight-%, more preferably at least 60 weight-%, more preferably at least 70 weight-%, more preferably at least 80 weight-%, more preferably at least 90 weight-%, more preferably at least 95 weight-%, more preferably at least 98 weight-%, more preferably at least 99 weight-% of the zeolitic MWW framework of the zeolitic material of the present invention consist of silicon, oxygen, and titanium.

Generally, the porous titanium-containing zeolitic material, wherein the framework structure of the titanium-containing zeolitic material comprised in the catalyst is the MWW framework structure, of the present invention may comprise at least one extra-zeolitic framework element. Conceivable extra-zeolitic framework elements include, but are no restricted to, Al, Zr, V, Nb, Ta, Cr, Mo, W, Mn, Fe, Co, Ni, Zn, Ga, Ge, In, Sn, Pb. Preferably, the porous titanium-containing zeolitic material of the present invention is essentially free of aluminum. The term "essentially free of aluminum" as used in this context of the present invention relates to a porous titanium-containing zeolitic material which comprises 500 ppm or less aluminum, preferably, 300 ppm or less aluminum, more preferably 200 ppm or less aluminum based on the total weight of the titanium-containing zeolitic material. Therefore, more preferred extra-zeolitic framework elements are selected from the group consisting of Zr, V, Nb, Ta, Cr, Mo, W, Mn, Fe, Co, Ni, Zn, Ga, Ge, In, Sn, Pb, and combinations of two or more thereof. According to a conceivable embodiment, the extra-zeolitic framework element includes, preferably is, Zn.

If the porous titanium-containing zeolitic material, wherein the framework structure of the titanium-containing zeolitic material comprised in the catalyst is the MWW framework structure, of the present invention comprises at least one extra-zeolitic framework element, the at least one element is preferably comprised in an amount in the range of from 0.1 to 10 weight-%, more preferably from 0.2 to 7 weight-%, more preferably from 0.5 to 5 weight-%, based on the total weight of the titanium-containing zeolitic material and regarding the sum of all extra-zeolitic framework elements

comprised in the titanium-containing zeolitic material.

As mentioned above, the framework structure of the titanium-containing zeolitic materials comprised in the catalyst is MWW.

Therefore, the present invention also relates to a process for oxidizing a sulfoxide to the respective sulfone, said process comprising

(i) reacting the sulfoxide with hydrogen peroxide in the presence of a catalyst, obtaining a mixture (M) comprising the sulfone and the catalyst,

wherein the catalyst comprises a titanium-containing zeolitic material having framework structure MWW as the catalytically active material.

Therefore, the present also relates to a process for oxidizing the sulfoxide of formula (III)

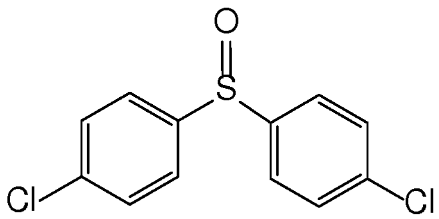

(III)

to the respective sulfone of formula (IV)

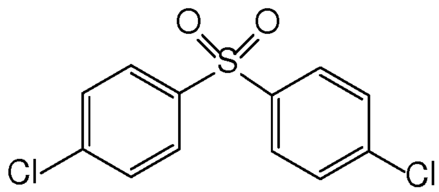

(IV),

said process comprising

(i) reacting the sulfoxide with hydrogen peroxide in the presence of a catalyst, obtaining a mixture (M) comprising the sulfone and the catalyst,

wherein the catalyst comprises a titanium-containing zeolitic material having framework structure MWW as the catalytically active material.

Therefore, the present also relates to a process for oxidizing the sulfoxide of formula (IIIa)

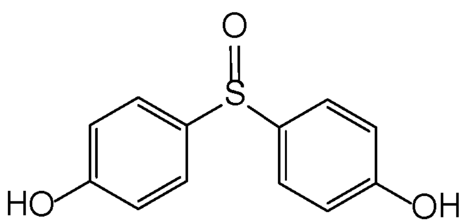

(IIIa)

to the respective sulfone of formula (IV)

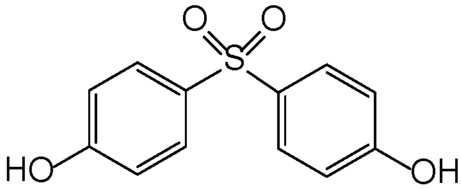

(IVa),

said process comprising

(i) reacting the sulfoxide with hydrogen peroxide in the presence of a catalyst, obtaining a mixture (M) comprising the sulfone and the catalyst,

wherein the catalyst comprises a titanium-containing zeolitic material having framework structure MWW as the catalytically active material.

Preferably, the MWW framework structure of the titanium-containing zeolitic material comprised in the catalyst has a titanium content in the range of from 0.5 to 3.0 weight-%, preferably from 1 to 2.5 weight-%, more preferably from 1.2 to 2.2 weight-%, calculated as element and based on the total weight of the titanium-containing zeolitic material, and a silicon content in the range of from 30 to 50 weight-%, preferably from 35 to 48 weight-%, more preferably from 38 to 47 weight-%, calculated as element and based on the total weight of the titanium-containing zeolitic material. Therefore, as mentioned above, the titanium-containing zeolitic material comprised in the catalyst used in the process of the present invention is a titanium-containing zeolitic material having an MWW framework structure, hereinafter referred to as TiMWW.

Thus, the present invention also relates to a process for oxidizing a sulfoxide to the respective sulfone, said process comprising

(i) reacting the sulfoxide with hydrogen peroxide in the presence of a catalyst, obtaining a mixture (M) comprising the sulfone and the catalyst,

wherein the catalyst comprises TiMWW as a catalytically active material, preferably as the catalytically active material, wherein the MWW framework structure has a titanium content in the range of from 0.5 to 3.0 weight-%, preferably from 1 to 2.5 weight-%, more preferably from 1.2 to 2.2 weight-%, calculated as element and based on the total weight of the TiMWW.

Further, the present invention relates to a process for oxidizing a sulfoxide to the respective sulfone, said process comprising

(i) reacting the sulfoxide with hydrogen peroxide in the presence of a catalyst, obtaining a mixture (M) comprising the sulfone and the catalyst,

wherein the catalyst comprises TiMWW as a catalytically active material, preferably as the catalytically active material, wherein the MWW framework structure has a titanium content in the range of from 0.5 to 3.0 weight-%, preferably from 1 to 2.5 weight-%, more preferably from 1.2 to 2.2 weight-%, calculated as element and based on the total weight of the TiMWW, and wherein at least 95 weight-%, preferably at least 98 weight-%, more preferably at least 99 weight-%, more preferably at least 99.5 weight-%, more preferably at least 99.9 weight% of the the MWW framework structure of the titanium-containing zeolitic material comprised in the catalyst consist of Ti, Si, O, and H.

Therefore, the present also relates to a process for oxidizing the sulfoxide of formula (III)

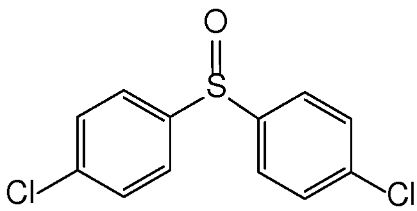

(III)

to the respective sulfone of formula (IV)

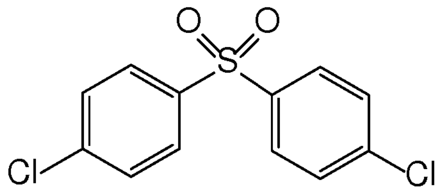

(IV),

said process comprising

(i) reacting the sulfoxide with hydrogen peroxide in the presence of a catalyst, obtaining a mixture (M) comprising the sulfone and the catalyst,

wherein the catalyst comprises TiMWW as a catalytically active material, preferably as the catalytically active material, wherein the MWW framework structure has a titanium content in the range of from 0.5 to 3.0 weight-%, preferably from 1 to 2.5 weight-%, more preferably from 1.2 to 2.2 weight-%, calculated as element and based on the total weight of the TiMWW, and wherein at least 95 weight-%, preferably at least 98 weight-%, more preferably at least 99 weight-%, more preferably at least 99.5 weight-%, more preferably at least 99.9 weight-% of the the MWW framework structure of the titanium-containing zeolitic material comprised in the catalyst consist of Ti, Si, O, and H.

Therefore, the present also relates to a process for oxidizing the sulfoxide of formula (IIIa)

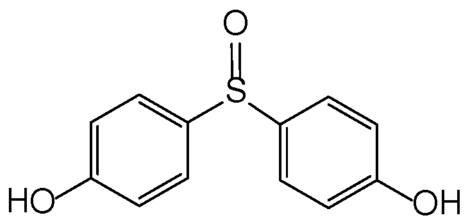

(IIIa)

to the respective sulfone of formula (IV)

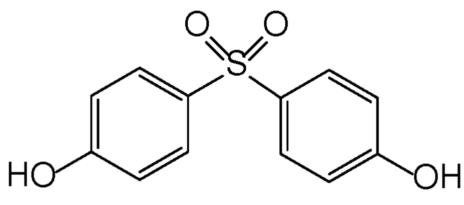

(IVa),

said process comprising

(i) reacting the sulfoxide with hydrogen peroxide in the presence of a catalyst, obtaining a mixture (M) comprising the sulfone and the catalyst,

wherein the catalyst comprises TiMWW as a catalytically active material, preferably as the catalytically active material, wherein the MWW framework structure has a titanium content in the range of from 0.5 to 3.0 weight-%, preferably from 1 to 2.5 weight-%, more preferably from 1.2 to 2.2 weight-%, calculated as element and based on the total weight of the TiMWW, and wherein at least 95 weight-%, preferably at least 98 weight-%, more preferably at least 99 weight-%, more preferably at least 99.5 weight-%, more preferably at least 99.9 weight-% of the the MWW framework structure of the titanium-containing zeolitic material comprised in the catalyst consist of Ti, Si, O, and H.

The TiMWW may comprise at least one extra-zeolitic framework element. Conceivable extra-zeolitic framework elements include, but are no restricted to, Al, Zr, V, Nb, Ta, Cr, Mo, W, Mn, Fe, Co, Ni, Zn, Ga, Ge, In, Sn, Pb. Preferably, the TiMWW is essentially free of aluminum. The term "essentially free of aluminum" as used in this context of the present invention relates to a TiMWW which comprises 500 ppm or less aluminum, preferably, 300 ppm or less aluminum, more

preferably 200 ppm or less aluminum based on the total weight of the TiMWW. Therefore, more preferred extra-zeolitic framework elements are selected from the group consisting of Zr, V, Nb, Ta, Cr, Mo, W, Mn, Fe, Co, Ni, Zn, Ga, Ge, In, Sn, Pb, and combinations of two or more thereof. According to a conceivable embodiment, the extra-zeolitic framework element includes, preferably is, Zn.

Preferably, the MWW framework structure of the titanium-containing zeolitic material comprised in the catalyst comprises at most 0.08 weight-% of boron, preferably at most 0.05 weight-% weight-% of boron, calculated as element and based on the total weight of the titanium-containing zeolitic material.

Preferred process for the preparation of TiMWW

Preferably, a zeolitic material of structure type MWW containing titanium (TiMWWW) is prepared in a first step, wherein the obtained TiMWW is optionally subjected in a second step to a suitable treatment to obtain ZnTiMWW.

It is preferred that the TiMWW, optionally further containing zinc, is prepared according to a process comprising

(I) preparing an aluminum-free zeolitic material of structure type MWW containing boron (B-MWW);
(II) deboronating the B-MWW to obtain an aluminum-free zeolitic material of structure type MWW (MWW);
(III) incorporating titanium (Ti) into the MWW to obtain an aluminum-free zeolitic material of structure type MWW containing Ti (TiMWW);
(IV) preferably acid-treating the TiMWW.

Stage (I)

As far as (I) is concerned, no specific restrictions exist. Preferably, a suitable starting mixture, preferably an aqueous mixture, containing preferably a B containing source and the Si containing source, preferably including at least one suitable micropore-forming agent, is subjected to hydrothermal crystallization under autogenous pressure. For crystallization purposes, it may be conceivable to use at least one suitable seeding material. As suitable Si containing precursors, fumed silica or colloidal silica, preferably colloidal silica such as ammonia-stabilized colloidal silica such as Ludox® AS-40 may be mentioned by way of example. As suitable boron containing precursor, boric acid, $B_2O_3$, borate salts, preferably boric acid may be mentioned by way of example. As suitable micropore-forming agent, piperidine, hexamethylene imine, or mixtures of piperidine and hexamethylene imine may be mentioned by way of example. Preferably, the crystallization time is in the range of from 3 to 8 days, more preferably from 4 to 6 days. During hydrothermal synthesis, the crystallization mixture may be stirred. The temperatures applied during crystallization are preferably in the range of from 160 to 200 °C, more preferably from 160 to 180 °C. The B-MMW precursor is obtained in its mother liquor, wherein the mother liquor has preferably a pH above 9.

Preferably, after hydrothermal synthesis, the pH of the mother liquor containing the obtained crystalline zeolitic material B-MMW precursor is adjusted, preferably to a value in the range of from 6 to 9.

The obtained crystalline zeolitic material B-MWW precursor is preferably suitably separated from the mother liquor. All methods of separating the B-MWW precursor from its mother liquor are conceivable. These methods include, for example, filtration, ultrafiltration, diafiltration and centrifugation methods or, for instance, spray drying processes and spray granulation processes. A combination of two or more of these methods can be applied. According to the present invention, the B-MWW precursor is preferably separated from its mother liquid by filtration to obtain a filter cake which is preferably subjected to washing, preferably with water. Subsequently, the filter cake, optionally further processed to obtain a suitable suspension, is subjected to spray drying or to ultrafiltration. Prior to separating the B-MWW precursor from its mother liquor, it is possible to increase the B-MWW precursor content of the mother liquor by concentrating the suspension. If washing is applied, it is preferred to continue the washing process until the washing water has a conductivity of less than 1,000 microSiemens/cm, more preferably of less than 900 microSiemens/cm, more preferably of less than 800 microSiemens/cm, more preferably of less than 700 microSiemens/cm.

After separation of the B-MWW from the suspension, preferably achieved via filtration, and after washing, the washed filter cake containing the B-MWW precursor is preferably subjected to pre-drying, for example by subjecting the filter cake to a suitable gas stream, preferably a nitrogen stream, for a time preferably in the range of from 4 to 10 h, more preferably from 5 to 8 h.

Subsequently, the pre-dried filter cake is preferably dried at temperatures in the range of from 100 to 300 °C, more preferably from 150 to 275 °C, more preferably from 200 to 250 °C in a suitable atmosphere such as technical nitrogen, air, or lean air, preferably in air or lean air. Such drying can be accomplished, for example, by spray-drying. Further, it is possible to separate the B-MWW precursor from its mother liquor via a suitable filtration method, followed by washing and spray-drying.

After drying, the B-MWW precursor is preferably subjected to calcination to obtain the B-MWW at temperatures in the range of from 500 to 700 °C, more preferably from 550 to 675 °C, more preferably from 600 to 675 °C in a suitable

atmosphere such as technical nitrogen, air, or lean air, preferably in air or lean air.

Stage (II)

As far as (II) is concerned, no specific restrictions exist. Preferably, the deboration of the B-MWW to obtain the zeolitic material of structure type MWW (MWW) is achieved via suitable treatment of the B-MWW with a liquid solvent system which may or may not contain at least one inorganic and/or at least one organic acid, or a salt thereof. Conceivable acids are, for example, hydrochloric acid, sulfuric acid, nitric acid, phosphoric acid, formic acid, acetic acid, propionic acid, oxalic acid, and tartaric acid. Preferred acids are inorganic acids, with nitric acid being especially preferred. The liquid solvent system is preferably selected from the group consisting of water, monohydric alcohols, polyhydric alcohols, and mixtures of two or more thereof.

Preferably, the liquid solvent system is selected from the group consisting of water, monohydric alcohols, polyhydric alcohols, and mixtures of two or more thereof, and wherein said liquid solvent system does not contain an inorganic or organic acid or a salt thereof, the acid being selected from the group consisting of hydrochloric acid, sulfuric acid, nitric acid, phosphoric acid, formic acid, acetic acid, propionic acid, and tartaric acid. More preferably, the liquid solvent system does not contain an inorganic or organic acid, or a salt thereof. Even more preferably, the liquid solvent system is selected from the group consisting of water, methanol, ethanol, propanol, ethane-1,2-diol, propane-1,2-diol, propane-1,3-diol, propane-1,2,3-triol, and mixtures of two or more thereof. Most preferably, the liquid solvent system is water.

The treatment according to (II) is preferably carried out at a temperature in the range of from 75 to 125 °C, more preferably from 85 to 115 °C, for a time preferably in the range of from 8 to 15 h, more preferably from 9 to 12 h.

The obtained deboronated crystalline zeolitic material MWW is preferably suitably separated from the suspension further comprising water and/or acid. All methods of separating the MWW from the suspension are conceivable. These methods include, for example, filtration, ultrafiltration, diafiltration and centrifugation methods or, for instance, spray drying processes and spray granulation processes. A combination of two or more of these methods can be applied. According to the present invention, the MWW is preferably separated from the suspension by filtration to obtain a filter cake which is preferably subjected to washing, preferably with water. Subsequently, the filter cake, optionally further processed to obtain a suitable suspension, is subjected to spray drying or to ultrafiltration. Prior to separating the MWW from the suspension, it is possible to increase the MWW content of the suspension by concentrating the suspension. If washing is applied, it may be preferred to continue the washing process until the washing water has a conductivity of less than 1,000 microSiemens/cm, more preferably of less than 900 microSiemens/cm, more preferably of less than 800 microSiemens/cm, more preferably of less than 700 microSiemens/cm.

After separation of the MWW from the suspension, preferably achieved via filtration, and after washing, the washed filter cake containing the MWW is preferably subjected to pre-drying, for example by subjecting the filter cake to a suitable gas stream, preferably a nitrogen stream, for a time preferably in the range of from 4 to 10 h, more preferably from 5 to 8 h.

Subsequently, the pre-dried filter cake is preferably dried at temperatures in the range of from 100 to 300 °C, more preferably from 150 to 275 °C, more preferably from 200 to 250 °C in a suitable atmosphere such as technical nitrogen, air, or lean air, preferably in air or lean air. Such drying can be accomplished, for example, by spray-drying. Further, it is possible to separate the MWW from the suspension via a suitable filtration method, followed by washing and spray-drying.

After drying, the MWW can be subjected to calcination at temperatures in the range of from 500 to 700 °C, more preferably from 550 to 675 °C, more preferably from 600 to 675 °C in a suitable atmosphere such as technical nitrogen, air, or lean air, preferably in air or lean air. Preferably, no calcination is carried out according to (II).

Stage (III)

As far as (III) is concerned, no specific restrictions exist. Preferably, a suitable starting mixture, preferably an aqueous mixture, containing the MWW and a Ti containing precursor, and preferably containing at least one suitable micropore-forming agent, is subjected to hydrothermal crystallization under autogenous pressure. It may be conceivable to use at least one suitable seeding material. As suitable Ti containing precursor, tetraalkylorthotitanates such as tetrabutylorthotitanate may be mentioned by way of example. As suitable micropore-forming agent, piperidine, hexamethylene imine, or mixtures of piperidine and hexamethylene imine may be mentioned by way of example. Preferably, the crystallization time is in the range of from 4 to 8 days, more preferably from 4 to 6 days. During hydrothermal synthesis, the crystallization mixture may be stirred. The temperatures applied during crystallization are preferably in the range of from 160 to 200 °C, more preferably from 160 to 180 °C.

After hydrothermal synthesis, the obtained crystalline zeolitic material TiMWW is preferably suitably separated from the mother liquor. All methods of separating the TiMWW from its mother liquor are conceivable. These methods

include, for example, filtration, ultrafiltration, diafiltration and centrifugation methods or, for instance, spray drying processes and spray granulation processes. A combination of two or more of these methods can be applied. According to the present invention, the TiMWW is preferably separated from its mother liquid by filtration to obtain a filter cake which is preferably subjected to washing, preferably with water. Subsequently, the filter cake, optionally further processed to obtained a suitable suspension, is subjected to spray drying or to ultrafiltration. Prior to separating the TiMWW from its mother liquor, it is possible to increase the TiMWW content of the mother liquor by concentrating the suspension. If washing is applied, it is preferred to continue the washing process until the washing water has a conductivity of less than 1,000 microSiemens/cm, more preferably of less than 900 microSiemens/cm, more preferably of less than 800 microSiemens/cm, more preferably of less than 700 microSiemens/cm.

After separation of the TiMWW from its mother liquor, preferably achieved via filtration, and after washing, the washed filter cake containing the TiMWW is preferably subjected to pre-drying, for example by subjecting the filter cake to a suitable gas stream, preferably a nitrogen stream, for a time preferably in the range of from 4 to 10 h, more preferably from 5 to 8 h.

Subsequently, the pre-dried filter cake is preferably dried at temperatures in the range of from 100 to 300 °C, more preferably from 150 to 275 °C, more preferably from 200 to 250 °C in a suitable atmosphere such as technical nitrogen, air, or lean air, preferably in air or lean air. Such drying can be accomplished, for example, by spray-drying to obtain a spray-powder.

In the alternative, the TiMWW is preferably not separated form the mother liquor following the hydrothermal synthesis. Thus, it is preferred that the mother liquor comprising the TiMWW obtained in the hydrothermal synthesis is directly subjected to spray-drying to obtain a spray-powder.

After drying, the TiMWW may be subjected to calcination at temperatures in the range of from 500 to 700 °C, more preferably from 550 to 675 °C, more preferably from 600 to 675 °C in a suitable atmosphere such as technical nitrogen, air, or lean air, preferably in air or lean air. Preferably, no calcination is carried out according to (III).

Stage (IV)

Stage (IV) of the process of the present invention preferably serves for reducing the Ti content of the TiMWW as obtained from stage (III), which reduction of the Ti content is preferably achieved by the acid treatment, and preferably also for reducing the carbon content, which reduction of the carbon content is preferably achieved by the calcination as described below. It is noted that according to a conceivable embodiment of the present invention, it may be possible to prepare a TiMWW in stage (III) which already exhibits the desired Ti content. Further, it may be possible in stage (III) to carry out a suitable calcination which results in a carbon content which is low enough so that the respectively obtained TiMWW could be processed further according to stage (V).

Generally, as far as (IV) is concerned, no specific restrictions exist. Preferably, the acid treatment of the TiMWW as obtained according to stage (III) to obtain the finally desired aluminum-free zeolitic material of structure type TiMWW is achieved via suitable treatment of the TiMWW with at least one acid, preferably an inorganic acid, more preferably nitric acid. The treatment according to (IV) is preferably carried out at a temperature in the range of from 75 to 125 °C, more preferably from 85 to 115 °C, for a time preferably in the range of from 17 to 25 h, more preferably from 18 to 22 h.

After the acid treatment, the obtained crystalline zeolitic material TiMWW is preferably suitably separated from the suspension further comprising an acid. All methods of separating the TiMWW from the suspension are conceivable. These methods include, for example, filtration, ultrafiltration, diafiltration and centrifugation methods or, for instance, spray drying processes and spray granulation processes. A combination of two or more of these methods can be applied. According to the present invention, the TiMWW is preferably separated from the suspension by filtration to obtain a filter cake which is preferably subjected to washing, preferably with water. Subsequently, the filter cake, optionally further processed to obtained a suitable suspension, is subjected to spray drying or to ultrafiltration. Prior to separating the TiMWW from the suspension, it is possible to increase the TiMWW content of the suspension by concentrating the suspension. If washing is applied, it may be preferred to continue the washing process until the washing water has a conductivity of less than 1,000 microSiemens/cm, more preferably of less than 900 microSiemens/cm, more preferably of less than 800 microSiemens/cm, more preferably of less than 700 microSiemens/cm.

After separation of the TiMWW from the suspension, preferably achieved via filtration, and after washing, the washed filter cake containing the TiMWW is preferably subjected to pre-drying, for example by subjecting the filter cake to a suitable gas stream, preferably a nitrogen stream, for a time preferably in the range of from 4 to 10 h, more preferably from 5 to 8 h.

Subsequently, the pre-dried filter cake is preferably dried at temperatures in the range of from 100 to 300 °C, more preferably from 150 to 275 °C, more preferably from 200 to 250 °C in a suitable atmosphere such as technical nitrogen, air, or lean air, preferably in air or lean air. Such drying can be accomplished, for example, by spray-drying to obtain a spray-powder. Further, it is possible to separate the TiMWW from the suspension via a suitable filtration method, followed by washing and spray-drying.

After drying, the TiMWW is preferably subjected to calcination at temperatures in the range of from 500 to 700 °C, more preferably from 550 to 675 °C, more preferably from 600 to 675 °C in a suitable atmosphere such as technical nitrogen, air, or lean air, preferably in air or lean air.

The TiMWW obtained in stage (IV) preferably has a titanium content in the range of from 0.5 to 3.0 weight-%, preferably from 1 to 2.5 weight-%, more preferably from 1.2 to 2.2 weight-%, calculated as element and based on the total weight of the titanium-containing zeolitic material, and a silicon content in the range of from 30 to 50 weight-%, preferably from 35 to 48 weight-%, more preferably from 38 to 47 weight-%, calculated as element and based on the total weight of the titanium-containing zeolitic material.

Conceivable Stage (V)

According to stage (V), the TiMWW preferably obtained according to stage (IV) may be optionally subjected to a suitable Zn treatment. Generally, as far as (V) is concerned, no specific restrictions exist provided that above-defined preferred ZnTiMWW can be obtained having the preferred Zn and Ti content. Most preferably, stage (V) comprises at least one suitable impregnation stage, more preferably at least one wet impregnation stage.

Concerning this impregnation stage, it is preferred to contact the TiMWW preferably as obtained according to (IV) is contacted with at least one suitable Zn-containing precursor in at least one suitable solvent (wet impregnation), most preferably water. As suitable Zn-containing precursor, water-soluble Zn salts are especially preferred, with zinc acetate dihydrate being especially preferred. It is further preferred to prepare a solution of the Zn-containing precursor, preferably an aqueous solution, and to suspend the TiMWW in this solution. Further preferably, impregnation is carried out at elevated temperatures, relative to room temperature, preferably in the range of from 75 to 125 °C, more preferably from 85 to 115 °C, for a time preferably in the range of from 3.5 to 5 h, more preferably from 3 to 6 h. Stirring the suspension during impregnation is preferred. After the impregnation, the obtained ZnTiMWW is preferably suitably separated from the suspension. All methods of separating the ZnTiMWW from the suspension are conceivable. Especially preferably, separation is carried out via filtration, ultrafiltration, diafiltration or centrifugation methods. A combination of two or more of these methods can be applied. According to the present invention, the ZnTiMWW is preferably separated from the suspension by filtration to obtain a filter cake which is preferably subjected to washing, preferably with water. If washing is applied, it may be preferred to continue the washing process until the washing water has a conductivity of less than 1,000 microSiemens/cm, more preferably of less than 900 microSiemens/cm, more preferably of less than 800 microSiemens/cm, more preferably of less than 700 microSiemens/cm. Subsequently, the preferably washed filter cake is subjected to pre-drying, for example by subjecting the filter cake to a suitable gas stream, preferably a nitrogen stream, for a time preferably in the range of from 5 to 15 h, more preferably from 8 to 12. Preferably, the ZnTiMWW obtained from the impregnation in (V) has a zinc content preferably in the range of from 1.0 to 2.0 weight-%, more preferably from 1.1 to 1.7 weight-%, more preferaby from 1.2 to 1.6 weight-%, more preferably from 1.3 to 1.5 weight-%, calculated as elemental zinc, a titanium content in the range of from 0.5 to 3.0 weight-%, preferably from 1 to 2.5 weight-%, more preferably from 1.2 to 2.2 weight-%, calculated as element and based on the total weight of the titanium-containing zeolitic material, and a silicon content in the range of from 30 to 50 weight-%, preferably from 35 to 48 weight-%, more preferably from 38 to 47 weight-%, calculated as element and based on the total weight of the titanium-containing zeolitic material.

Spray powder and molding

The reacting according to step (i) of the process of the present invention can be carried out, for example, in batch mode, in semi-continuous mode, and/or in continuous mode. Depending on the respective mode, it is possible to employ the porous titanium-containing silicate, preferably the titanium-containing zeolitic material, more preferably the TiMWW, as powder. Preferably, if the TiMWW is employed as porous titanium-containing silicate, it is possible to employ the TiMWW as powder, preferably as spray-powder, as obtained according to stage (IV) as described hereinabove.

Preferably, the catalyst used in the process of the present invention is a spray-powder. Optionally, the spray-powder is contained in a molding wherein the molding preferably comprises at least one binder, preferably a silica binder.

Preferably, at least 95 weight-%, preferably at least 98 weight-%, more preferably at least 99 weight-% of the spray powder consist of the porous titanium-containing silicate, preferably the titanium-containing zeolitic material.

Preferably, the spray-powder is present in the form of particles which have a Dv10 value in the range of from 3 to 10 micrometer, preferably from 4 to 6 micrometer, a Dv50 value in the range of from 7 to 50 micrometer, preferably from 8 to 30 micrometer and a Dv90 value in the range of from 12 to 90 micrometer, preferably from 13 to 70 micrometer.

Preferably, the spray powder comprises mesopores having an average pore diameter (4V/A) in the range of from 10 to 50 nm, preferably from 15 to 45 nm, as determined by Hg porosimetry according to DIN 66133, and comprising macropores having an average pore diameter (4V/A) in the range of from more than 50 nanometer preferably in the range of from 0.06 to 3 micrometer, as determined by Hg porosimetry according to DIN 66133.

Generally, it is possible to employ the spray powder according to the present invention as such, without any further modifications as a catalyst for the process of the present invention.

It is also possible that based on the spray-powder, a molding is prepared containing the spray-powder. In such a process, the spray-powder, optionally after further modification, is suitably shaped and optionally post-treated. Such further modification of the spray-powder may comprise impregnation of the spray-powder with a solution containing at least one heteroatom, thereby incorporating at least one heteroatom, optionally followed by drying and/or calcining. The molding may be suitably post-treated by incorporating at least one noble metal and/or by subjecting the molding to a water-treatment, wherein the water-treatment comprises treating the molding with liquid water in an autoclave under autogenous pressure at elevated temperatures, followed by optional drying and/or calcination of the molding.

For preparing a molding, the spray-powder used as catalyst in the process of the present invention can be admixed with at least one binder and/or with at least one binder precursor, and optionally with at least one pore-forming agent and/or at least one plasticizing agent.

Examples of suitable binders are metal oxides, such as, for example, $SiO_2$, $Al_2O_3$, $TiO_2$, $ZrO_2$ or MgO or clays or mixtures of two or more of these oxides or mixed oxides of at least two of Si, Al, Ti, Zr, and Mg. Clay minerals and naturally occurring or synthetically produced alumina, such as, for example, alpha-, beta-, gamma-, delta-, eta-, kappa-, chi- or theta-alumina and their inorganic or organometallic precursor compounds, such as, for example, gibbsite, bayerite, boehmite or pseudoboehmite or trialkoxyaluminates, such as, for example, aluminum triisopropylate, are particularly preferred as $Al_2O_3$ binders. Further conceivable binders might be amphiphilic compounds having a polar and a non-polar moiety and graphite. Further binders might be, for example, clays, such as, for example, montmorillonites, kaolins, metakaoline, hectorite, bentonites, halloysites, dickites, nacrites or anaxites. Silica binders are especially preferred.

The moldings used in the process of the present invention may contain, based on the weight of the moldings, up to 95 weight-% or up to 90 weight-% or up to 85 weight-% or up to 80 weight-% or up to 75 weight-% or up to 70 weight-% or up to 65 weight-% or up to 60 weight-% or up to 55 weight-% or up to 50 weight-% or up to 45 weight-% or up to 40 weight-% or up to 35 weight-% or up to 30 weight-% or up to 25 weight-% or up to 20 weight-% or up to 15 weight-% or up to 10 weight-% or up to 5 weight-% of one or more binder materials. Preferably, the moldings of the present invention contain from 10 to 50 weight-%, preferably from 15 to 40 weight-%, more preferably from 20 to 30 weight-% binder, most preferably a silica binder.

Pore forming agents include, but are not limited to, polymers such as polymeric vinyl compounds, such as polyalkylene oxides like polyethylene oxides, polystyrene, polyacrylates, polymethacrylates, polyolefins, polyamides and polyesters, carbohydrates, such as cellulose or cellulose derivatives like methyl cellulose, or sugars or natural fibers. Further suitable pore forming agents may be, for example, pulp or graphite. If desired with regard to the pore characteristics be achieved, a mixture of two or more pore forming agents may be used.

Plasticizing agents include organic, in particular hydrophilic polymers, such as carbohydrate like cellulose, cellulose derivatives, such as methyl cellulose, and starch such as potato starch, wallpaper plaster, polyacrylates, polymethacrylates, polyvinyl alcohol, polyvinylpyrrolidone, polyisobutene or polytetrahydrofuran. The use of water, alcohols or glycols or mixtures thereof, such as mixtures of water and alcohol, or water and glycol, such as for example water and methanol, or water and ethanol, or water and propanol, or water and propylenglycol, as plasticizing agents may be mentioned.

As to the geometry of the moldings used in the process of the present invention, no specific restrictions exist. In particular, the respective geometry may be chosen depending on the specific needs of the specific use of the moldings. When using the molding as catalyst, geometries such as strands, for example having rectangular, triangular hexagonal, quadratic, oval, or circular cross-section, stars, tablets, spheres, hollow cylinders, and the like are possible. One of the preferred geometries of the moldings of the present invention is a strand having circular cross-section. Such geometries are preferred if the moldings of the present invention are employed, for example, as fixed-bed catalysts, most preferably in a continuous-type reaction. The diameter of these strands having circular cross-section which can be prepared, e.g., via extrusion processes, is preferably in a range of from 1 to 4 mm, more preferably from 1 to 3 mm, more preferably from 1 to 2 mm, more preferably from 1.5 to 2 mm, more preferably from 1.5 to 1.7 mm.

For the moldings as catalysts such as fixed-bed catalysts, most preferably in a continuous-type reaction, it is generally necessary that the moldings have superior mechanic resistance in order to allow for a long-term use in the reactor. The molding used in the process of the present invention, preferably in the form of strands having circular cross-section and a diameter of from 1.5 to 1.7 mm, have a crush strength of the least 5 N, preferably a crush strength of up to 20 N, such as from 10 to 20 N, in particular from 11 to 20 N.

Hydrogen peroxide

According to the present invention, it is conceivable that the hydrogen peroxide which is used as oxidizing agent is formed in situ during the reaction from hydrogen and oxygen or from other suitable precursors.

Preferably, the hydrogen peroxide is not formed in situ but employed as starting material, preferably in the form

of a solution. Preferably, the hydrogen peroxide used in (i) is employed as an aqueous hydrogen peroxide solution. It is further preferred that the aqueous hydrogen peroxide solution has a hydrogen peroxide content in the range of from 10 to 70 weight-%, more preferably from 25 to 60 weight-%, more preferably from 20 to 50 weight-%, based on the total weight of the aqueous solution.

For the preparation of the hydrogen peroxide employed in (i), the anthraquinone process may be used. This process is based on the catalytic hydrogenation of an anthraquinone compound to form the corresponding anthrahydrochinone compound, subsequent reaction of this with oxygen to form hydrogen peroxide and subsequent extraction of the hydrogen peroxide formed. The cycle is completed by rehydrogenation of the anthraquinone compound which has been formed again in the oxidation. A review of the antraquinone process is given in "Ullmanns Encyclopedia of Industrial Chemistry", 5th edition, volume 13, pages 447 to 456.

It is also possible to prepare the hydrogen peroxide by anodic oxidation of sulfuric acid with simultaneous evolution of hydrogen at the cathode to produce peroxodisulfuric acid. Hydrolysis of the peroxodisulfuric acid forms firstly peroxosulfuric acid and then hydrogen peroxide and sulfuric acid, which is thus recovered.

Reacting in (i)

Preferably, at the beginning of the reaction in (i), the molar ratio of hydrogen peroxide relative to sulfoxide is in the range of from 1:1 to 50:1, more preferably from 2:1 to 30:1, more preferably from 3:1 to 10:1.

Preferably, at the beginning of the reaction in (i), the molar ratio of sulfoxide relative to titanium contained in the titanium-containing silicate, preferably in the framework structure of the titanium-containing zeolitic material, is in the range of from 10:1 to 500:1, more preferably from 30:1 to 300:1, more preferably form 50:1 to 200:1.

Generally, it is conceivable that the reacting in (i) is carried out in the absence of a solvent. Preferably, the reacting in (i) is carried out in the presence of a solvent.

Generally, there are no specific restrictions regarding the chemical nature of the solvent provided that the reacting in (i) can be carried out. Preferably, the solvent is a polar aprotic solvent. More preferably, the solvent is selected from the group consisting of 1-methyl-2-pyrrolidone, tetrahydrofuran, dioxane, chlorinated hydrocarbons, and a mixture of two or more thereof. Chlorinated hydrocarbons are preferably selected from the group consisting of dichloromethane, trichloromethane, trichloroethane, 1,2-dichloroethane, 1,1,2,2-tetrachloroethane, trichloroethylene, 1,2-dichlorobenzene, 1,2,4-trichlorobenzene, and a mixture of two or more thereof. More preferably, the solvent is 1-methyl-2-pyrrolidone.

Consequently, when the reacting in (i) is carried out in the presence of a solvent, the mixture (M) obtained from (i) additionally comprises the solvent.

Preferably, at beginning of the reacting according to (i), the molar ratio of sulfoxide relative to solvent is in the range of from 0.01: 1 to 10:1, preferably from 0.1:1 to 5:1, more preferably from 0.3:1 to 1:1.

Preferably, the reacting according to (i) is carried out in the presence of at least one inert gas. It is conceivable that an inert gas atmosphere comprising at least one inert gas is established above the liquid level at the beginning of the reaction according to (i), whereby no further inert gas is introduced during the reacting in (i). It is also conceivable that the at least one inert gas is introduced continuously into the liquid phase with a suitable flow rate for preferably at least at least partially during the reacting according to (i).

The term "inert gas" as used in this context of the present invention refers to a gas which does not, or not essentially, unfavorably interact with the starting materials, intermediate products or reaction products in the reaction mixture. More preferably, the inert gas is selected from the group consisting of nitrogen, helium, neon, argon, carbon dioxide, and a mixture of two or more thereof. More preferably, the inert gas is nitrogen, more preferably technical nitrogen.

Preferably, the reacting according to (i) is carried out at a temperature in the range of from 0 to 90 °C, more preferably from 2 to 85 °C, more preferably from 5 to 80 °C. A preferred temperature range is from 0 to 20 °C, preferably from 2 to 15 °C, more preferably from 5 to 10 °C. A further preferred temperature range is from 65 to 90 °C, preferably from 70 to 85 °C, more preferably from 75 to 80 °C. Yet a further preferred temperature range is from 30 to 60 °C, preferaby from 35 to 55 °C, more preferably from 40 to 50 °C. It is generally conceivable that during the reaction, two or more suitable different temperatures are applied, provided that these two or more temperatures are within above-mentioned preferred ranges. Heating and/or cooling during the process may be carried out continuously, semi-continuously, or discontinuously. The individual starting materials may be preheated before being mixed together or may be heated following after the mixing.

Preferably, the reaction according to (i) is carried out under a pressure of at most 15 bar, preferably at most 10 bar. It is generally conceivable that during the reacting in (i), two or more suitable different pressures are applied, provided that these two or more pressures are within above-mentioned preferred ranges. Increasing or decreasing the pressure during the process may be carried out continuously, semi-continuously, or discontinuously.

The reacting according to (i) is preferably carried out in batch mode, semicontinuous mode or in continuous mode.

The term "at the beginning of the reaction" as used in the context of the present invention refers to the starting

point of the process of the present invention. In batch mode, the "term at the beginning of the reaction" defines the time point at which all educts and the catalyst are present in the educt mixture. In continuous mode which is carried out in a suitable reactor, the "term at the beginning of the reaction" defines the point in the reactor downstream the reactor entrance where the starting materials and the catalyst are for the first time contacted with each other.

The sulfoxide is preferably subjected in (i) in a suitable reactor. Usually, the reactor comprises the heterogeneous catalyst arranged therein and is equipped with means for controlling the reaction pressure, the stirring rate, the inert gas flow, the temperature, and the like. The reactor is further suitable equipped with feeding and removal means. The reactor may be made of materials which are inert under reaction conditions. By way of example, glass or stainless steel may be mentioned.

Preferably, in continuous mode, the catalyst is present in the form of moldings, preferably in the form of strands, arranged in a suitable reactor, for example in the form of a fixed-bed, which enables a thorough contacting with the starting materials which are passed over the catalyst.

Preferably, in batch mode, the titanium-containing zeolitic material as catalytically active material is preferably present as a powder, preferably as a spray-powder, suspended in the liquid starting mixture. The contacting between the zeolitic material and the starting mixture may be enhanced by stirring.

A possible reduction of the activity of the titanium-containing zeolitic material in the course of the oxidation reaction, in particular when performed in continuous mode, may be compensated by adjusting the reaction temperature, pressure, stirring rate, and the like. Indicators for the activity of the titanium-containing zeolitic material are the conversion rate of the educts and the selectivity for the desired product. Conversion rate and selectivity may be calculated according to the formulas indicated below Table 1. Conversion rate and selectivity may be calculated based on the amounts of educts and products present in the reaction mixture at a given time point. The product and educt amounts may be determined by any suitable technique, e.g. chromatography.

When carrying out the reaction according to (i) in a batch mode, it is preferred that the reaction is carried out for a period of time in the range of from 1 to 15 h, preferably from 2 to 10 h, more preferably from 4 to 6 h.

The mixture (M) obtained in (i) following the reaction of sulfoxide with hydrogen peroxide in the presence of a catalyst comprises a sulfone and the catalyst and optionally a solvent. It is conceivable that the mixture (M) obtained in (i) further comprises unreacted starting material and/or one or more by-products.

Step (ii)

It is preferred that in an additional step (ii), the catalyst is separated from the mixture (M).

The separation of the catalyst may be achieved by any conceivable method. Preferably, in particular in case the reacting in (i) is carried out in batch mode, the catalyst is separated by filtration, centrifugation, draining of mixture (M), pumping out the mixture (M), or a suitable combination of two or more of these methods. More preferably, the catalyst comprising the titanium-containing zeolitic material is separated from the mixture (M) by filtration.

It is conceivable that downstream of the separation of the catalyst according to step (ii), the mixture (M) is directly used, for example without separating the sulfone contained in the mixture (M), as starting material in a suitable reaction. A suitable reaction includes, but is not limited to, a polymerization reaction, wherein the sulfone comprised in mixture (M) is reacted, for example, with one or more suitable compounds such as one or more bifunctional nucleophilic compounds. A suitable polymerization reaction may comprise the preparation of a polyethersulfone such as poly(oxy-1,4-phenylsulfonyl-1,4-phenyl).

Step (iii)

Preferably, the sulfone contained in the mixture (M), preferably the sulfone according to formula (II) contained in the mixture (M), is separated from the mixture (M) in a step (iii).

Preferably, the sulfone, preferably the sulfone according to formula (II), is separated from the mixture (M) by precipitation, crystallization, extraction, solvent evaporation, or a suitable combination of two or more thereof. More preferably the sulfone, preferably the sulfone according to formula (II), is separated from the mixture (M) by precipitation.

It is possible that the sulfone, preferably the sulfone according to formula (II), obtained by separation from mixture (M) is submitted to further purification steps. Further purifications steps may be selected from recrystallization, chromatography, sublimation, or a suitable combination of two or more thereof.

The process of the present invention has considerable advantages over the preparations of sulfones according to the prior art.

In numerous processes of the prior art, oxidizing agents such as peracetic acid and homogeneous catalysts such as Lewis acids are used. Using these compounds require considerable safety precautions so that the sulfone production, particularly at large scale, becomes complex and cost-intensive. Also, by using such acidic compounds considerable amounts of waste water are generated which requires a thorough regeneration before its release in the

environment. A further disadvantage of using a homogeneous catalyst is its time- and energy-consuming separation from the product mixture. The process of the present invention has none of these disadvantages, since a substantially inert, heterogeneous catalyst comprising a porous titanium-containing silicate is used, which may be easily separated from the reaction mixture.

Further, the methods of the prior art using heterogeneous catalysts are considerably more complex than the process of the present invention, the former requiring several subsequent reaction stages under highly specific conditions. The educt mixtures of these methods are also complex, further requiring the presence of several different strong acidic compounds in stoichiometric amounts. However, the process of the present invention using a heterogeneous catalyst comprising a porous titanium-containing silicate may be carried out in one single step under constant conditions by reacting merely two educts, a sulfoxide and hydrogen peroxide, to obtain the desired sulfone at favorable yields.

The present invention is further defined by the following embodiments and the combination of embodiments characterized by the respective dependencies:

1. A process for oxidizing a sulfoxide to the respective sulfone, said process comprising

    (i) reacting the sulfoxide with hydrogen peroxide in the presence of a catalyst, obtaining a mixture (M) comprising the sulfone and the catalyst,
    wherein the catalyst comprises a porous titanium-containing silicate as a catalytically active material and wherein the porous titanium-containing silicate comprised in the catalyst is a titanium-containing zeolitic material having a zeolitic framework structure comprising titanium and silicon, wherein the framework structure of the titanium-containing zeolitic material comprised in the catalyst is the MWW framework structure.

2. The process of embodiment 1, wherein the sulfoxide has a structure according to formula (I)

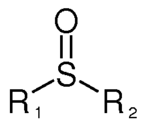 (I),

and the respective sulfone has a structure according to formula (II)

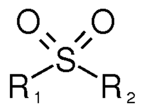 (II),

wherein $R_1$ and $R_2$ are independently from one another linear or branched, substituted or unsubstituted alkyl residues preferably having from 1 to 20 carbon atoms, linear or branched, substituted or unsubstituted alkenyl residues preferably having from 2 to 20 carbon atoms, or substituted or unsubstituted aryl or heteroaryl residues preferably having from 5 to 20 carbon atoms.

3. The process of embodiment 2, wherein $R_1$ and $R_2$ are independently from one another substituted or unsubstituted aryl residues, preferably substituted aryl residues.

4. The process of embodiment 3, wherein the substituents of the aryl residues are selected from the group consisting of halogen, preferably F, Cl, Br, or I, hydroxyl, linear or branched alkyl residues preferably having from 1 to 10 carbon atoms, linear or branched alkyloxy residues preferably having from 1 to 10 carbon atoms, linear or branched alkenyl residues preferably having from 2 to 10 carbon atoms, aryl residues preferably having from 5 to 10 carbon atoms, heteroaryl residues preferably having from 5 to 10 carbon atoms, and combinations of two or more thereof, wherein the heteroatoms of the heteroaryl residues are preferably selected from the group consisting of N, P, O, and S.

5. The process of any of embodiments 2 to 4, wherein $R_1$ and $R_2$ are independently from one another substituted aryl residues having from 5 to 10 carbon atoms, preferably from 6 to 10 carbon atoms, wherein the substituents are preferably halogen, more preferably F, Cl, Br, or I, or hydroxyl, more preferably Cl or hydroxyl.

6. The process of any of embodiments 2 to 5, wherein $R_1$ and $R_2$ are independently from one another substituted aryl residues having 6, wherein the substituents are Cl or hydroxyl.

7. The process of any of embodiments 1 to 6, wherein the sulfoxide is 4,4'-dichlorodiphenylsulfoxide.

8. The process of any of embodiments 1 to 6, wherein the sulfoxide is 4,4'-dihydroxydiphenylsulfoxide.

9. The process of embodiment 1, wherein the titanium-containing zeolitic material comprised in the catalyst further comprises one or more elements selected from the group consisting of Al, Zr, V, Nb, Ta, Cr, Mo, W, Mn, Fe, Co, Ni, Zn, Ga, Ge, In, Sn, Pb and a mixture of two or more thereof, the further element preferably being Zn.

10. The process of embodiment 1 or 9, wherein the framework structure of the titanium-containing zeolitic material comprised in the catalyst has a titanium content in the range of from 0.5 to 3.0 weight-%, preferably from 1.0 to 2.5 weight-%, more preferably from 1.2 to 2.2 weight-%, calculated as element and based on the total weight of the titanium-containing zeolitic material.

11. The process of any of embodiments 1 or 9 to 10, wherein the framework structure of the titanium-containing zeolitic material comprised in the catalyst has a and a silicon content in the range of from 30 to 50 weight-%, preferably from 35 to 48 weight-%, more preferably from 38 to 47 weight-%, calculated as element and based on the total weight of the titanium-containing zeolitic material.

12. The process of any of embodiments 11 or 9 to 11, wherein the framework structure of the titanium-containing zeolitic material comprised in the catalyst comprises boron in an amount of from 0 to 0.08 weight-% of boron, preferably from 0 to 0.05 weight-%, calculated as element and based on the total weight of the titanium-containing zeolitic material.

13. The process of any of embodiments 1 or 9 to 12, wherein the framework structure of the titanium-containing zeolitic material comprised in the catalyst is the MWW framework structure, wherein the framework structure of the titanium-containing zeolitic material comprised in the catalyst has a titanium content in the range of from 0.5 to 3.0 weight-%, calculated as element and based on the total weight of the titanium-containing zeolitic material, and wherein the framework structure of the titanium-containing zeolitic material comprised in the catalyst has a silicon content in the range of from 30 to 50 weight-%, calculated as element and based on the total weight of the titanium-containing zeolitic material.

14. The process of any of embodiments 1 or 9 to 13, wherein the framework structure of the titanium-containing zeolitic material comprised in the catalyst is the MWW framework structure, wherein the framework structure of the titanium-containing zeolitic material comprised in the catalyst has a titanium content in the range of from 1.0 to 2.5 weight-%, calculated as element and based on the total weight of the titanium-containing zeolitic material, and wherein the framework structure of the titanium-containing zeolitic material comprised in the catalyst has a silicon content in the range of from 35 to 48 weight-%, calculated as element and based on the total weight of the titanium-containing zeolitic material.

15. The process of any of embodiments 1 or 9 to 14, wherein the framework structure of the titanium-containing zeolitic material comprised in the catalyst is the MWW framework structure, wherein the framework structure of the titanium-containing zeolitic material comprised in the catalyst has a titanium content in the range of from 1.2 to 2.2 weight-%, calculated as element and based on the total weight of the titanium-containing zeolitic material, and wherein the framework structure of the titanium-containing zeolitic material comprised in the catalyst has a silicon content in the range of from 38 to 47 weight-%, calculated as element and based on the total weight of the titanium-containing zeolitic material.

16. The process of any of embodiments13 to 15, wherein at least 95 weight-%, preferably at least 98 weight-%, more preferably at least 99 weight-%, more preferably at least 99.5 weight-%, more preferably at least 99.9 weight-% of the the MWW framework structure of the titanium-containing zeolitic material comprised in the catalyst consist of Ti, Si, O, and H.

17. The process of any of embodiments 1 to 16, wherein the hydrogen peroxide used in (i) is employed as an aqueous hydrogen peroxide solution, preferably having a hydrogen peroxide content in the range of from 10 to 70 weight-%, more preferably from 15 to 60 weight-%, more preferably from 20 to 50 weight-%, based on the total weight of the aqueous solution.

18. The process of any of embodiments 1 to 17, wherein at the beginning of the reaction according to (i), the molar

ratio of hydrogen peroxide relative to sulfoxide is in the range of from 1:1 to 50:1, preferably from 2:1 to 30:1, more preferably from 3:1 to 10:1.

19. The process of any of embodiments 1 to 18, wherein at the beginning of the reaction according to (i), the molar ratio of sulfoxide relative to titanium contained in the titanium-containing silicate, preferably in the framework structure of the titanium-containing zeolitic material, is in the range of from 10:1 to 500:1, preferably from 30:1 to 300:1, more preferably form 50:1 to 200:1.

20. The process of any of embodiments 1 to19, wherein the reaction according to (i) is carried out in the presence of a solvent and wherein the mixture (M) additionally comprises the solvent.

21. The process of embodiment 20, wherein the solvent is selected from the group consisting of 1-methyl-2-pyrrolidone, tetrahydrofuran, dioxane, chlorinated hydrocarbons, and a combination of two or more thereof.

22. The process of embodiment 20 or 21, wherein at the beginning of the reaction according to (i), the molar ratio of sulfoxide relative to solvent is in the range of from 0.01:1 to 10:1, preferably from 0.1:1 to 5:1, more preferably from 0.3:1 to 1:1.

23. The process of any of embodiments 1 to 22, wherein the reaction according to (i) is carried in the presence of at least one inert gas.

24. The process of embodiment 23, wherein the inert gas is selected from the group consisting of nitrogen, helium, neon, argon, carbon dioxide, and a mixture of two or more thereof, wherein the inert gas more preferably comprises nitrogen, more preferably comprises, more preferably consists of, technical nitrogen.

25. The process of any of embodiments 1 to 24, wherein the reaction according to (i) is carried out at a temperature in the range of from 0 to 90 °C, preferably from 2 to 85 °C, more preferably from 5 to 80 °C.

26. The process of any of embodiments 1 to 25, wherein the reaction according to (i) is carried out under a pressure of at most 15 bar, preferably at most 10 bar.

27. The process of any of embodiments 1 to 26, wherein the reaction according to (i) is carried out under a pressure in the range of from 1 to 15 bar, preferably from 1 to 10 bar.

28. The process of any of embodiments 1 to 27, wherein the reaction according to (i) is carried out in batch mode.

29. The process of embodiment 28, wherein the reaction according to (i) is carried out for a period of time in the range of from 1 to 15 h, preferably from 2 to 10 h, more preferably from 4 to 6 h.

30. The process of any of embodiments 1 to 27, wherein the reaction according to (i) is carried out in continuous mode.

31. The process of any of embodiments 1 to 30, further comprising

(ii) separating the catalyst from the mixture (M), preferably by filtration.

32. The process of any of embodiments 1 to 31, further comprising

(iii) separating the sulfone according to formula (II) from the mixture (M), preferably by precipitation.

33. The process of any of embodiments 1 to 32, wherein the catalyst is a spray-powder.

34. The process of embodiment 33, wherein at least 95 weight-%, preferably at least 98 weight-%, more preferably at least 99 weight-% of the spray powder consist of the titanium-containing silicate, preferably the titanium-containing zeolitic material.

35. The process of embodiment 33 or 34, wherein the spray-powder is contained in a molding.

36. The process of embodiment 35, wherein the molding comprises, in addition to the spray-powder, at least one

binder, preferably a silica binder.

The present invention is further illustrated by the following examples.

## Examples

### Example 1: Preparation of a titanium containing zeolitic material having an MWW framework structure (Ti-MWW)

### Example 1.1 Synthesis of the boron-containing MWW (B-MWW)

a) Hydrothermal synthesis

480 kg de-ionized water were provided in a vessel. Under stirring at 70 rpm (rounds per minute), 166 kg boric acid were suspended in the water. The suspension was stirred for another 3 h. Subsequently, 278 kg piperidine were added, and the mixture was stirred for another hour. To the resulting solution, 400 kg Ludox® AS-40 were added, and the resulting mixture was stirred at 70 rpm for another hour.

In this synthesis mixture, the boron source boric acid, calculated as elemental boron, relative to the silicon source Ludox® AS-40, calculated as elemental silicon, was present in a molar ratio of 1:1; the water relative to the silicon source Ludox® AS-40, calculated as elemental silicon, was present in a molar ratio of 10:1; and the template compound piperidine relative to the silicon source Ludox® AS-40, calculated as elemental silicon, was present in a molar ratio of 1.2:1.

The finally obtained mixture was transferred to a crystallization vessel and heated to 175 °C within 5 h under autogenous pressure and under stirring (50 rpm). The temperature of 175 °C was kept essentially constant for 60 h; during these 60 h, the mixture was stirred at 50 rpm. Subsequently, the mixture was cooled to a temperature of from 50-60 °C within 5 h.

The mother liquor containing the crystallized BMWW precursor had a pH of 11.3 as determined via measurement with a pH electrode.

b) pH adjustment

To the mother liquor obtained in a), 1400 kg of a 10 weight-% $HNO_3$ aqueous solution were added under stirring at 50 rpm (rounds per minute). The adding was carried out at a temperature of the suspension of 40 °C. After the addition of the 10 weight-% $HNO_3$ aqueous solution, the resulting suspension was further stirred for 5 h under stirring at 50 rpm at a temperature of the suspension of 40 °C. The pH of the thus pH-adjusted mother liquor as determined via measurement with a pH electrode was 7.

c) Spray-drying and calcination

From the pH-adjusted mother liquor obtained in b), the B-MWW precursor was separated by filtration using different types of filtration devices (suction filter with filter material Sefar Tetex® Mono 24-1100-SK 012, centrifugal filter, candle filter). The filter cake was then washed with de-ionized water until the washing water had a conductivity of less then 700 microSiemens/cm. From the washed filter cake, an aqueous suspension was prepared having a solids content of 15 weight-%. The suspension was subjected to spray-drying in a spray-tower with the following spray-drying conditions:

| | | |
|---|---|---|
| drying gas, nozzle gas: | | technical nitrogen |
| temperature drying gas: | | |
| - | temperature spray tower (in): | 270-340 °C |
| - | temperature spray tower (out): | 150-167 °C |
| - | temperature filter (in): | 140-160 °C |
| - | temperature scrubber (in): | 50-60 °C |
| - | temperature scrubber (out): | 34-36 °C |
| pressure difference filter: | | 8.3-10.3 mbar |
| nozzle: | | |
| - | two-component nozzle | supplier Gerig; size 0 |
| - | nozzle gas temperature: | room temperature |
| - | nozzle gas pressure: | 2.5 bar |
| operation mode: | | nitrogen straight |

(continued)

| | |
|---|---|
| apparatus used: | spray tower with one nozzle |
| configuration: | spray tower - filter - scrubber |
| gas flow: | 1900 kg/h |
| filter material: | Nomex® needle-felt 20 $m^2$ |
| dosage via flexible tube pump: | SP VF 15 (supplier: Verder) |

The spray tower was comprised of a vertically arranged cylinder having a length of 2,650 mm, a diameter of 1,200 mm, which cylinder was conically narrowed at the bottom. The length of the conus was 600 mm. At the head of the cylinder, the atomizing means (a two-component nozzle) were arranged. The spray-dried material was separated from the drying gas in a filter downstream of the spray tower, and the drying gas was then passed through a scrubber. The suspension was passed through the inner opening of the nozzle, and the nozzle gas was passed through the ring-shaped slit encircling the opening.

The spray-dried material was then subjected to calcination at 650 °C in a rotary calciner with a throughput in the range of from 0.8 to 1.0 kg/h.

The obtained BMWW had a boron content of 1.3 weight-%, a silicon content of 44 weight-%, and a total organic carbon (TOC) content of less than 0.1 weight-% and a crystallinity of 88 %, determined by XRD. The BET specific surface area determined via nitrogen adsorption at 77 K according to DIN 66131 was 468 $m^2$/g.

**Example 1.2: Preparation of deboronated zeolitic material having an MWW framework structure**

a) Deboronation

1590 kg water was passed into a vessel equipped with a reflux condenser. Under stirring at 40 rpm, 106 kg of the spray-dried material obtained according to section 1.1 were suspended into the water. Subsequently, the vessel was closed and the reflux condenser put into operation. The stirring rate was increased to 70 rpm under stirring at 70 rpm, the content of the vessel was heated to 100 °C within 10 h and kept at this temperature for 10 h. Then, the content of the vessel was cooled to a temperature of less than 50 °C. The resulting deboronated zeolitic material of structure type MWW was separated from the suspension by filtration under a nitrogen pressure of 2.5 bar and washed four times with deionized water. After the filtration, the filter cake was dried in a nitrogen stream for 6 h. The obtained deboronated zeolitic had a residual moisture content of 80 %, as determined using an IR (infrared) scale at 160 °C.

b) Spray-drying

From the nitrogen-dried filter cake having a residual moisture content of 80 % obtained according to section a) above, an aqueous suspension was prepared with deionized water, the suspension having a solid content of 15 weight-%. This suspension was subjected to spray-drying in a spray-tower with the following spray-drying conditions:

| | | |
|---|---|---|
| drying gas, nozzle gas: | | technical nitrogen |
| temperature drying gas: | | |
| temperature spray tower (in): | | 290-310 °C |
| - | temperature spray tower (out): | 140-160 °C |
| - | temperature filter (in): | 140-160 °C |
| - | temperature scrubber (in): | 40-60 °C |
| - | temperature scrubber (out): | 20-40 °C |
| pressure difference filter: | | 6.0-10.0 mbar |
| nozzle: | | |
| - | two-component nozzle: | supplier Niro, diameter 4 mm |
| - | nozzle gas pressure: | 2.5 bar |
| operation mode: | | nitrogen straight |
| apparatus used: | | spray tower with one nozzle |
| configuration: | | spray tower - filter - scrubber |
| gas flow: | | 1900 kg/h |
| filter material: | | Nomex® needle-felt 20 $m^2$ |
| dosage via flexible tube pump: | | VF 15 (supplier: Verder) |

The spray tower was comprised of a vertically arranged cylinder having a length of 2,650 mm, a diameter of 1,200 mm, which cylinder was conically narrowed at the bottom. The length of the conus was 600 mm. At the head of the cylinder, the atomizing means (a two-component nozzle) were arranged. The spray-dried material was separated from the drying gas in a filter downstream of the spray tower, and the drying gas was then passed through a scrubber. The suspension was passed through the inner opening of the nozzle, and the nozzle gas was passed through the ring-shaped slit encircling the opening. The obtained spray-dried zeolitic material having an MWW framework structure had a boron content of 0.04 weight-%, a silicon content of 42 weight-%, a total organic carbon (TOC) content of less than 0.1 weight-%, and a crystallinity of 82 %, determined a by XRD. The BET specific surface area determined via nitrogen adsorption at 77 K according to DIN 66131 was 462 $m^2$/g.

**Example 1.3 Preparation of a titanium containing zeolitic material having an MWW framework structure**

a) Hydrothermal synthesis

Based on the deboronated MWW material obtained above, a zeolitic material of structure type MWW containing titanium (Ti) was prepared, referred to in the following as TiMWW.

| | | |
|---|---|---|
| Starting materials: | deionized water: | 789 g |
| | piperidine: | 291 g |
| | tetrabutylorthotitanate: | 41.4 g |
| | deboronated zeolitic material: | 192 g |

500 g of distilled water was filled in a beaker and 291g piperidine were added and the mixture was stirred for 5 min. Afterwards 41.,4 g of tetrabutylorthotitanate was added under stirring and the mixture was further stirred for 30 min before the addition of 289 g of distilled water. After stirring for another 10 min, 192 g of MWW material were added under stirring and the suspension was further stirred for another 30 min. The suspension was then transferred to an autoclave and heated in 90 min to 170 °C under stirring (100 rpm) and kept there for 48 h. The pressure increase during the synthesis is 9 bar. Subsequently, the obtained suspension containing TiMWW was cooled within 1 h below 50 °C.

b) Spray-drying

The obtained suspension was diluted with water to have a concentration of water of 85 weight-% directly subjected to spray-drying in a spray-tower with the following spray-drying conditions:

| | | |
|---|---|---|
| drying gas, nozzle gas: | | technical nitrogen |
| temperature drying gas: | | |
| - | temperature spray tower (in): | 160-200 °C |
| - | temperature spray tower (out): | 150-170 °C |
| - | temperature filter (in): | 150-170 °C |
| - | temperature scrubber (in): | 30-50 °C |
| - | temperature scrubber (out): | 30-50 °C |
| pressure difference filter: | | 6.0-10.0 mbar |
| nozzle: | | |
| - | top-component nozzle: | supplier Niro, diameter 4 mm |
| - | nozzle gas pressure: | 1,5 bar |
| operation mode: | | nitrogen straight |
| apparatus used: | | spray tower with one nozzle |
| configuration: | | spray tower - filter - scrubber |
| gas flow: | | 1800 kg/h |
| filter material: | | PE with PTF Membrane , Surface 1,13 $m^2$ |
| dosage via flexible tube pump: | | SP VF 15 (supplier: Verder) |

The spray tower was comprised of a vertically arranged cylinder having a length of 2,650 mm, a diameter of 1,200 mm, which cylinder was conically narrowed at the bottom. The length of the conus was 600 mm. At the head of the cylinder, the atomizing means (a two-component nozzle) were arranged. The spray-dried material was separated

from the drying gas in a filter downstream of the spray tower, and the drying gas was then passed through a scrubber. The suspension was passed through the inner opening of the nozzle, and the nozzle gas was passed through the ring-shaped slit encircling the opening.

**Example 1.4 Acid treatment of the titanium containing zeolitic material having an MWW framework (TiMWW)**

a) Acid treatment

The spray-dried TiMWW material as obtained above was subjected to acid treatment as described in the following:

| | | |
|---|---|---|
| Starting materials: | deionized water: | 1885 g |
| | nitric acid (65 %) (mixed with the water becomes 10 weight-%): | 365 g |
| | spray-dried TiMWW according to Example 1.3: | 50 g |

1885 g deionized water were filled in a vessel. 365 g nitric acid were added, and 50 g of the spray-dried TiMWW were added under stirring. The mixture in the vessel was heated to 100 °C and kept at this temperature under autogenous pressure for 1 h under stirring (250 rpm). The thus obtained mixture was then cooled within 1 h to a temperature of less than 50 °C. The cooled mixture was subjected to filtration, and the filter cake was washed with 4 L of water. After the filtration, the filter cake was dried in an oven at 120 °C for 10 h.

b) Calcination

The dried material was then subjected to calcination at 650 °C for 5 h (heating ramp 2K/min).

The calcined material had a silicon content of 44 weight-%, a titanium content of 1.7 weight-% and a total organic carbon content of less than 0.1 weight-%. The Langmuir surface are determined via nitrogen adsorption at 77 K according to DIN 66131 was 584 $m^2/g$, the multipoint BET specific surface area determined via nitrogen adsorption at 77 K according t DIN 66131 was 432 $m^2/g$. The degree of crystallization determined via XRD was 84 %, the average crystallite size 29.0 nm.

**Example 2: Oxidation of 4,4'-Dichlorodiphenylsulfoxide (DCDPSO) with $H_2O_2$ by use of a TiMWW obtained according to Example 1**

In a glass autoclave cooled with ice, 0.5 g of the TiMWW obtained according to Example 1 (1.4) were introduced, followed by the addition of a separately prepared solution of 60 g 1-methyl-2-pyrolidone and 5.0 g DCDPSO (commercially available from Sigma-Aldrich, CAS 3085-42-5). This corresponds to about 0.8 weight-% TiMWW catalyst relative to the total amount of the obtained suspension. After the addition of the reactants the autoclave was closed and flushed with nitrogen. The suspension was stirred with a magnetic stirrer at 700 rpm and the autoclave was heated to 8 °C. When the autoclave temperature reached the reaction temperature of 8 °C, 10 g of an aqueous hydrogen peroxide solution (35 weight-% in water) was pumped into autoclave. After the addition of hydrogen peroxide the reaction mixture was continuously stirred for 5 hours. Subsequently, the autoclave was opened and the catalyst was removed by filtration and the reaction mixture was analyzed by GC and GC-MS.

The conversion rates of DCDPSO and the selectivity for DCDPS (4,4'-Dichlorodiphenylsulfone) in % obtained for Example 2 are summarized in Table 1 below. The conversion and selectivity were calculated according to the formulas indicated below Table 1 based on a GC analysis. A 30 m CP Sil 8 column with an internal diameter of 0.25 mm ID was used for analysis.

**Example 3: Oxidation of 4,4'-Dichlorodiphenylsulfoxide (DCDPSO) with $H_2O_2$ by use of a TiMWW obtained according to Example 1**

In a glass autoclave cooled with ice, 0.5 g of the TiMWW obtained according to Example 1 (1.4) were introduced, followed by the addition of a separately prepared solution of 60g 1-methyl-2-pyrolidone and 5.0 g DCDPSO (commercially available from Sigma-Aldrich, CAS 3085-42-5). This corresponds to about 0.8 weight-% TiMWW catalyst relative to the total amount of the obtained suspension. After the addition of the reactants the autoclave was closed and flushed with nitrogen. The suspension was stirred with a magnetic stirrer at 700 rpm and the autoclave was heated to 50 °C. When the autoclave temperature reached the reaction temperature of 50 °C, 10 g of an aqueous hydrogen peroxide solution (35 weight-% in water) was pumped into autoclave. After the addition of hydrogen peroxide the reaction mixture was

continuously stirred for 5 hours. Subsequently, the autoclave was opened and the catalyst was removed by filtration and the reaction mixture was analyzed by GC and GC-MS.

The conversion rates of DCDPSO and the selectivity for DCDPS (4,4'-Dichlorodiphenylsulfone) in % obtained for Example 3 are summarized in Table 1 below. The conversion and selectivity were calculated according to the formulas indicated below Table 1 based on a GC analysis. A 30 m CP Sil 8 column with an internal diameter of 0.25 mm ID was used for analysis.

**Example 4: Oxidation of 4,4'-Dichlorodiphenylsulfoxide (DCDPSO) with $H_2O_2$ by use of a TiMWW obtained according to Example 1**

In a glass autoclave cooled with ice, 0.5 g of the TiMWW obtained according to Example 1 (1.4) were introduced, followed by the addition of a separately prepared solution of 60 g 1-methyl-2-pyrolidone and 5.0 g DCDPSO (commercially available from Sigma-Aldrich, CAS 3085-42-5). This corresponds to about 0.8 weight-% TiMWW catalyst relative to the total amount of the obtained suspension. After the addition of the reactants the autoclave was closed and flushed with nitrogen. The suspension was stirred with a magnetic stirrer at 700 rpm and the autoclave was heated to 70 °C. When the autoclave temperature reached the reaction temperature of 70 °C, 10 g of an aqueous hydrogen peroxide solution (35 weight-% in water) was pumped into autoclave. After the addition of hydrogen peroxide the reaction mixture was continuously stirred for 5 hours. Subsequently, the autoclave was opened and the catalyst was removed by filtration and the reaction mixture was analyzed by GC and GC-MS.

The conversion rates of DCDPSO and the selectivity for DCDPS (4,4'-Dichlorodiphenylsulfone) in % obtained for Example 4 are summarized in Table 1 below. The conversion and selectivity were calculated according to the formulas indicated below Table 1 based on a GC analysis. A 30 m CP Sil 8 column with an internal diameter of 0.25 mm ID was used for analysis.

**Example 5: Oxidation of 4,4'-Dichlorodiphenylsulfoxide (DCDPSO) with $H_2O_2$ by use of a TiMWW obtained according to Example 1**

In a glass autoclave cooled with ice, 1.0 g of the TiMWW obtained according to Example 1 (1.4) were introduced, followed by the addition of a separately prepared solution of 60 g 1-methyl-2-pyrolidone and 5.0 g DCDPSO (commercially available from Sigma-Aldrich, CAS 3085-42-5). This corresponds to about 1.5 weight-% TiMWW catalyst relative to the total amount of the obtained suspension. After the addition of the reactants the autoclave was closed and flushed with nitrogen. The suspension was stirred with a magnetic stirrer at 700 rpm and the autoclave was heated to 70 °C. When the autoclave temperature reached the reaction temperature of 70 °C, 10 g of an aqueous hydrogen peroxide solution (35 weight-% in water) was pumped into autoclave. After the addition of hydrogen peroxide the reaction mixture was continuously stirred for 5 hours. Subsequently, the autoclave was opened and the catalyst was removed by filtration and the reaction mixture was analyzed by GC and GC-MS.

The conversion rates of DCDPSO and the selectivity for DCDPS (4,4'-Dichlorodiphenylsulfone) in % obtained for Example 5 are summarized in Table 1 below. The conversion and selectivity were calculated according to the formulas indicated below Table 1 based on a GC analysis. A 30 m CP Sil 8 column with an internal diameter of 0.25 mm ID was used for analysis.

**Example 6: Oxidation of 4,4'-Dichlorodiphenylsulfoxide (DCDPSO) with $H_2O_2$ by use of a TiMWW obtained according to Example 1**

In a glass autoclave cooled with ice, 1.0 g of the TiMWW obtained according to Example 1 (1.4) were introduced, followed by the addition of a separately prepared solution of 60 g 1-methyl-2-pyrolidone and 5.0 g DCDPSO (commercially available from Sigma-Aldrich, CAS 3085-42-5). This corresponds to about 0.8 weight-% TiMWW catalyst relative to the total amount of the obtained suspension. After the addition of the reactants the autoclave was closed and flushed with nitrogen. The suspension was stirred with a magnetic stirrer at 700 rpm and the autoclave was heated to 70 °C. When the autoclave temperature reached the reaction temperature of 70 °C, 7 g of an aqueous hydrogen peroxide solution (35 weight-% in water) was pumped into autoclave. After the addition of hydrogen peroxide the reaction mixture was continuously stirred for 5 hours. Subsequently, the autoclave was opened and the catalyst was removed by filtration and the reaction mixture was analyzed by GC and GC-MS.

The conversion rates of DCDPSO and the selectivity for DCDPS (4,4'-Dichlorodiphenylsulfone) in % obtained for Example 6 are summarized in Table 1 below. The conversion and selectivity were calculated according to the formulas indicated below Table 1 based on a GC analysis. A 30 m CP Sil 8 column with an internal diameter of 0.25 mm ID was used for analysis.

**Example 7: Oxidation of 4,4'-Dichlorodiphenylsulfoxide (DCDPSO) with $H_2O_2$ by use of a TiMWW obtained according to Example 1**

In a glass autoclave cooled with ice, 0.5 g of the TiMWW obtained according to Example 1 (1.4) were introduced, followed by the addition of a separately prepared solution of 60 g tetrahydrofuran and 5.0 g DCDPSO (commercially available from Sigma-Aldrich, CAS 3085-42-5). This corresponds to about 0.8 weight-% TiMWW catalyst relative to the total amount of the obtained suspension. After the addition of the reactants the autoclave was closed and flushed with nitrogen. The suspension was stirred with a magnetic stirrer at 700 rpm and the autoclave was heated to 50 °C. When the autoclave temperature reached the reaction temperature of 50 °C, 10 g of an aqueous hydrogen peroxide solution (35 weight-% in water) was pumped into autoclave. After the addition of hydrogen peroxide the reaction mixture was continuously stirred for 5 hours. Subsequently, the autoclave was opened and the catalyst was removed by filtration and the reaction mixture was analyzed by GC and GC-MS.

The conversion rates of DCDPSO and the selectivity for DCDPS (4,4'-Dichlorodiphenylsulfone) in % obtained for Example 7 are summarized in Table 1 below. The conversion and selectivity were calculated according to the formulas indicated below Table 1 based on a GC analysis. A 30 m CP Sil 8 column with an internal diameter of 0.25 mm ID was used for analysis.

**Table 1**

| | Temp. / °C | Amount of catalyst in reaction mixture / weight-% | Ratio DCDPSO: $H_2O$ | Time / h | Conversion rate DCDPSO / % | Selectivity DCDPS / % |
|---|---|---|---|---|---|---|
| | | | | | | |
| Example 2 | 8 | 0.8 | 0.2 | 5 | 64.4 | 82.5 |
| Example 3 | 50 | 0.8 | 0.2 | 5 | 47.5 | 98.6 |
| Example 4 | 70 | 0.8 | 0.2 | 5 | 42.6 | 87.2 |
| Example 5 | 70 | 1.5 | 0.2 | 5 | 77.8 | 92.1 |
| Example 6 | 70 | 0.8 | 0.2 | 5 | 48.0 | 85.3 |
| Example 7 | 50 | 0.8 | 0.2 | 5 | 16.2 | 71.6 |

The conversion rate of DCDPSO was calculated based on the following equation:

$$\text{Conversion (\%)} = 100 - (\text{mol(DCDPSO) after reaction / mol(DCDPSO) introduced}) * 100$$

The selectivity of DCDPS was calculated based on the following equation:

$$\text{Selectivity (\%)} = (\text{mol(DCDPS) after the reaction / mol(DCDPSO) consumed}) * 100$$

**Cited Prior Art**

- RU-C-2158257
- CN-A-102351757
- CN-A-102351756
- WO-A-2012/143281
- CN-A-102838516

- US-A-4287366
- US-A-2010/120869

## Claims

**1.** A process for oxidizing a sulfoxide to the respective sulfone, said process comprising

(i) reacting the sulfoxide with hydrogen peroxide in the presence of a catalyst, obtaining a mixture (M) comprising the sulfone and the catalyst,

wherein the catalyst comprises a porous titanium-containing silicate as a catalytically active material and wherein the porous titanium-containing silicate comprised in the catalyst is a titanium-containing zeolitic material having a zeolitic framework structure comprising titanium and silicon, wherein the framework structure of the titanium-containing zeolitic material comprised in the catalyst is the MWW framework structure.

**2.** The process of claim 1, wherein the sulfoxide has a structure according to formula (I)

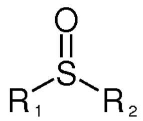

(I),

and the respective sulfone has a structure according to formula (II)

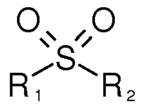

(II),

wherein $R_1$ and $R_2$ are independently from one another linear or branched, substituted or unsubstituted alkyl residues preferably having from 1 to 20 carbon atoms, linear or branched, substituted or unsubstituted alkenyl residues preferably having from 2 to 20 carbon atoms, or substituted or unsubstituted aryl or heteroaryl residues preferably having from 5 to 20 carbon atoms, wherein the sulfoxide is preferably 4,4'-dichlorodiphenylsulfoxide or 4,4'-dihydroxydiphenylsulfoxide.

**3.** The process of claim 1, wherein the framework structure of the titanium-containing zeolitic material comprised in the catalyst has a titanium content in the range of from 0.5 to 3.0 weight-%, preferably from 1.0 to 2.5 weight-%, more preferably from 1.2 to 2.2 weight-%, calculated as element and based on the total weight of the titanium-containing zeolitic material, and a silicon content in the range of from 30 to 50 weight-%, preferably from 35 to 48 weight-%, more preferably from 38 to 47 weight-%, calculated as element and based on the total weight of the titanium-containing zeolitic material.

**4.** The process of any of claims 1 to 3, wherein the hydrogen peroxide used in (i) is employed as an aqueous hydrogen peroxide solution, preferably having a hydrogen peroxide content in the range of from 10 to 70 weight-%, more preferably from 20 to 50 weight-%, based on the total weight of the aqueous solution, wherein at the beginning of the reaction according to (i), the molar ratio of hydrogen peroxide relative to sulfoxide is preferably in the range of from 1:1 to 50:1, more preferably from 2:1 to 30:1, more preferably from 3:1 to 10:1.

**5.** The process of any of claims 1 to 4, wherein at the beginning of the reaction according to (i), the molar ratio of sulfoxide relative to titanium contained in the titanium-containing silicate, preferably in the framework structure of the titanium-containing zeolitic material, is in the range of from 10:1 to 500:1, preferably from 30:1 to 300:1, more preferably form 50:1 to 200:1.

**6.** The process of any of claims 1 to 5, wherein the reaction according to (i) is carried out in the presence of a solvent and wherein the mixture (M) additionally comprises the solvent, wherein the solvent is preferably selected from the group consisting of 1-methyl-2-pyrrolidone, tetrahydrofuran, dioxane, chlorinated hydrocarbons, and a combination

of two or more thereof, and wherein at the beginning of the reaction according to (i), the molar ratio of sulfoxide relative to solvent is preferably in the range of from 0.01:1 to 10:1, more preferably from 0.1:1 to 5:1, more preferably from 0.3:1 to 1:1.

**7.** The process of any of claims 1 to 6, wherein the reaction according to (i) is carried in the presence of at least one inert gas.

**8.** The process of any of claims 1 to 7, wherein the reaction according to (i) is carried out at a temperature in the range of from 0 to 90 °C, preferably from 2 to 85 °C, more preferably from 5 to 80 °C.

**9.** The process of any of claims 1 to 8, wherein the reaction according to (i) is carried out under a pressure of at most 15 bar, preferably at most 10 bar.

**10.** The process of any of claims 1 to 9, wherein the reaction according to (i) is carried out in batch mode.

**11.** The process of claim 10, wherein the reaction according to (i) is carried out for a period of time in the range of from 1 to 15 h, preferably from 2 to 10 h, more preferably from 4 to 6 h.

**12.** The process of any of claims 1 to 11, further comprising

(ii) separating the catalyst from the mixture (M).

**13.** The process of any of claims 1 to 12, further comprising

(iii) separating the sulfone according to formula (II) from the mixture (M), preferably by precipitation.

**14.** The process of any of claims 1 to 13, wherein the catalyst is a spray-powder, optionally contained in a molding, the molding preferably comprising at least one binder, preferably a silica binder, wherein preferably at least 95 weight-%, more preferably at least 98 weight-%, more preferably at least 99 weight-% of the spray powder consist of the titanium-containing zeolitic material.

**Patentansprüche**

**1.** Verfahren zur Oxidation eines Sulfoxids zum entsprechenden Sulfon, wobei das Verfahren Folgendes umfasst:

(i) Umsetzen des Sulfoxids mit Wasserstoffperoxid in Gegenwart eines Katalysators, was eine Mischung (M) ergibt, die das Sulfon und den Katalysator umfasst,

wobei der Katalysator ein poröses titanhaltiges Silikat als katalytisch aktives Material umfasst und wobei es sich bei dem porösen titanhaltigen Silikat in dem Katalysator um ein titanhaltiges zeolithisches Material mit einer zeolithischen Gerüststruktur, die Titan und Silicium umfasst, handelt, wobei es sich bei der Gerüststruktur des titanhaltigen zeolithischen Materials in dem Katalysator um die MWW-Gerüststruktur handelt.

**2.** Verfahren nach Anspruch 1, wobei das Sulfoxid eine Struktur gemäß Formel (I)

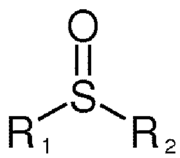

(I),

aufweist und das entsprechende Sulfon eine Struktur gemäß Formel (II)

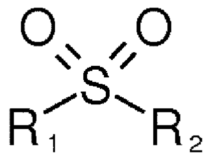

(II),

aufweist, wobei $R_1$ und $R_2$ unabhängig voneinander für lineare oder verzweigte, substituierte oder unsubstituierte Alkylreste mit vorzugsweise 1 bis 20 Kohlenstoffatomen, lineare oder verzweigte, substituierte oder unsubstituierte Alkenylreste mit vorzugsweise 2 bis 20 Kohlenstoffatomen oder substituierte oder unsubstituierte Aryl- oder Heteroarylreste mit vorzugsweise 5 bis 20 Kohlenstoffatomen stehen, wobei es sich bei dem Sulfoxid vorzugsweise um 4,4'-Dichlordiphenylsulfoxid oder 4,4'-Dihydroxydiphenylsulfoxid handelt.

**3.** Verfahren nach Anspruch 1, wobei die Gerüststruktur des titanhaltigen zeolithischen Materials in dem Katalysator einen Titangehalt im Bereich von 0,5 bis 3,0 Gew.-%, vorzugsweise von 1,0 bis 2,5 Gew.-%, weiter bevorzugt von 1,2 bis 2,2 Gew.-%, berechnet als Element und bezogen auf das Gesamtgewicht des titanhaltigen zeolithischen Materials, und einen Siliciumgehalt im Bereich von 30 bis 50 Gew.-%, vorzugsweise von 35 bis 48 Gew.-%, weiter bevorzugt von 38 bis 47 Gew.-%, berechnet als Element und bezogen auf das Gesamtgewicht des titanhaltigen zeolithischen Materials, aufweist.

**4.** Verfahren nach einem der Ansprüche 1 bis 3, wobei das in (i) verwendete Wasserstoffperoxid als wässrige Wasserstoffperoxidlösung, vorzugsweise mit einem Wasserstoffperoxidgehalt im Bereich von 10 bis 70 Gew.-%, weiter bevorzugt von 20 bis 50 Gew.-%, bezogen auf das Gesamtgewicht der wässrigen Lösung, eingesetzt wird, wobei zu Beginn der Umsetzung gemäß (i) das Molverhältnis von Wasserstoffperoxid zu Sulfoxid vorzugsweise im Bereich von 1:1 bis 50:1, weiter bevorzugt von 2:1 bis 30:1, weiter bevorzugt von 3:1 bis 10:1, liegt.

**5.** Verfahren nach einem der Ansprüche 1 bis 4, wobei zu Beginn der Umsetzung gemäß (i) das Molverhältnis von Sulfoxid zu Titan in dem titanhaltigen Silikat, vorzugsweise in der Gerüststruktur des titanhaltigen zeolithischen Materials, im Bereich von 10:1 bis 500:1, vorzugsweise von 30:1 bis 300:1, weiter bevorzugt von 50:1 bis 200:1, liegt.

**6.** Verfahren nach einem der Ansprüche 1 bis 5, wobei die Umsetzung gemäß (i) in Gegenwart eines Lösungsmittels durchgeführt wird und wobei die Mischung (M) zusätzlich das Lösungsmittel umfasst, wobei das Lösungsmittel vorzugsweise aus der Gruppe bestehend aus 1-Methyl-2-pyrrolidon, Tetrahydrofuran, Dioxan, chlorierten Kohlenwasserstoffen und einer Kombination von zwei oder mehr davon ausgewählt wird und wobei zu Beginn der Umsetzung gemäß (i) das Molverhältnis von Sulfoxid zu Lösungsmittel vorzugsweise im Bereich von 0,01:1 bis 10:1, weiter bevorzugt von 0,1:1 bis 5:1, weiter bevorzugt von 0,3:1 bis 1:1, beträgt.

**7.** Verfahren nach einem der Ansprüche 1 bis 6, wobei die Umsetzung gemäß (i) in Gegenwart mindestens eines Inertgases durchgeführt wird.

**8.** Verfahren nach einem der Ansprüche 1 bis 7, wobei die Umsetzung gemäß (i) bei einer Temperatur im Bereich von 0 bis 90 °C, vorzugsweise von 2 bis 85 °C, weiter bevorzugt von 5 bis 80 °C, durchgeführt wird.

**9.** Verfahren nach einem der Ansprüche 1 bis 8, wobei die Umsetzung gemäß (i) unter einem Druck von höchstens 15 bar, vorzugsweise höchstens 10 bar, durchgeführt wird.

**10.** Verfahren nach einem der Ansprüche 1 bis 9, wobei die Umsetzung gemäß (i) in diskontinuierlicher Fahrweise durchgeführt wird.

**11.** Verfahren nach Anspruch 10, wobei die Umsetzung gemäß (i) über einen Zeitraum im Bereich von 1 bis 15 h, vorzugsweise von 2 bis 10 h, weiter bevorzugt von 4 bis 6 h, durchgeführt wird.

**12.** Verfahren nach einem der Ansprüche 1 bis 11, ferner umfassend

(ii) Abtrennen des Katalysators aus der Mischung (M) .

**13.** Verfahren nach einem der Ansprüche 1 bis 12, ferner umfassend

(iii) Abtrennen des Sulfonats der Formel (II) aus der Mischung (M), vorzugsweise durch Ausfällung.

**14.** Verfahren nach einem der Ansprüche 1 bis 13, wobei es sich bei dem Katalysator um ein Sprühpulver handelt, das gegebenenfalls in einem Formkörper enthalten ist, wobei der Formkörper vorzugsweise mindestens ein Bindemittel, vorzugsweise mindestens ein Siliciumdioxid-Bindemittel, umfasst, wobei vorzugsweise mindestens 95 Gew.-%, weiter bevorzugt mindestens 98 Gew.-%, weiter bevorzugt mindestens 99 Gew.-%, des Sprühpulvers aus dem titanhaltigen zeolithischen Material bestehen.

## Revendications

**1.** Procédé d'oxydation d'un sulfoxyde en sulfone respective, ledit procédé comprenant

(i) la réaction du sulfoxyde avec du peroxyde d'hydrogène en présence d'un catalyseur, pour obtenir un mélange (M) comprenant la sulfone et le catalyseur,

dans lequel le catalyseur comprend un silicate contenant du titane poreux en tant que matériau catalytiquement actif et dans lequel le silicate contenant un titane poreux compris dans le catalyseur est un matériau zéolitique contenant du titane ayant une structure de charpente zéolitique comprenant du titane et du silicium, dans lequel la structure de charpente du matériau zéolitique contenant du titane compris dans le catalyseur est la structure de charpente MWW.

**2.** Procédé selon la revendication 1, dans lequel le sulfoxyde a une structure selon la formule (I)

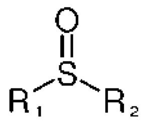

(I),

et la sulfone respective a une structure selon la formule (II)

- 2 -

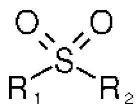

(II),

dans lesquelles $R_1$ et $R_2$ sont, indépendamment l'un de l'autre, des résidus alkyle substitués ou non substitués, linéaires ou ramifiés, ayant de préférence de 1 à 20 atomes de carbone, des résidus alcényle substitués ou non substitués, linéaires ou ramifiés ayant de préférence de 2 à 20 atomes de carbone, ou des résidus aryle ou hétéroaryle substitués ou non substitués ayant de préférence de 5 à 20 atomes de carbone, dans lequel le sulfoxyde est de préférence 4,4'-dichlorodiphénylsulfoxyde ou 4,4'-dihydroxydiphénylsulfoxyde.

**3.** Procédé selon la revendication 1, dans lequel la structure de charpente du matériau zéolitique contenant du titane compris dans le catalyseur a une teneur en titane dans la plage de 0,5 à 3,0 % en poids, de préférence de 1,0 à 2,5 % en poids, plus préférablement de 1,2 à 2,2 % en poids, calculée en élément et sur la base du poids total du matériau zéolitique contenant du titane, et une teneur en silicium dans la plage de 30 à 50 % en poids, de préférence de 35 à 48 % en poids, plus préférablement de 38 à 47 % en poids, calculée en élément et sur la base du poids total du matériau zéolitique contenant du titane.

**4.** Procédé selon l'une quelconque des revendications 1 à 3, dans lequel le peroxyde d'hydrogène utilisé dans (i) est utilisé sous la forme d'une solution aqueuse de peroxyde d'hydrogène, de préférence ayant une teneur en peroxyde d'hydrogène dans la plage de 10 à 70 % en poids, plus préférablement de 20 à 50 % en poids, sur la base du poids total de la solution aqueuse, dans lequel, au début de la réaction selon (i), le rapport molaire du peroxyde d'hydrogène au sulfoxyde est, de préférence, dans la plage de 1:1 à 50:1, plus préférablement de 2:1 à 30:1, plus préférablement de 3:1 à 10:1.

5. Procédé selon l'une quelconque des revendications 1 à 4, dans lequel, au début de la réaction selon (i), le rapport molaire du sulfoxyde au titane contenu dans le silicate contenant du titane, de préférence dans la structure de charpente du matériau zéolitique contenant du titane, est dans la plage de 10:1 à 500:1, de préférence de 30:1 à 300:1, plus préférablement de 50:1 à 200:1.

6. Procédé selon l'une quelconque des revendications 1 à 5, dans lequel la réaction selon (i) est conduite en présence d'un solvant et dans lequel le mélange (M) comprend en outre le solvant, le solvant étant de préférence choisi dans le groupe constitué des 1-méthyl-2-pyrrolidone, tétrahydrofurane, dioxane, hydrocarbures chlorés, et une combinaison de deux ou plus de ceux-ci, et dans lequel, au début de la réaction selon (i), le rapport molaire du sulfoxyde au solvant est de préférence dans la plage de 0,01:1 à 10:1, plus préférablement de 0,1:1 à 5:1, plus préférablement de 0,3:1 à 1:1.

7. Procédé selon l'une quelconque des revendications 1 à 6, dans lequel la réaction selon (i) est conduite en présence d'au moins un gaz inerte.

8. Procédé selon l'une quelconque des revendications 1 à 7, dans lequel la réaction selon (i) est conduite à une température dans la plage de de 0 à 90 °C, de préférence de 2 à 85 °C, plus préférablement de 5 à 80 °C.

9. Procédé selon l'une quelconque des revendications 1 à 8, dans lequel la réaction selon (i) est conduite sous une pression d'au plus 15 bar, de préférence au plus 10 bar.

10. Procédé selon l'une quelconque des revendications 1 à 9, dans lequel la réaction selon (i) est conduite en mode discontinu.

11. Procédé selon la revendication 10, dans lequel la réaction selon (i) est conduite pendant une durée pendant une durée dans la plage de 1 à 15 h, de préférence de 2 à 10 h, plus préférablement de 4 à 6 h.

12. Procédé selon l'une quelconque des revendications 1 à 11, comprenant en outre (ii) la séparation du catalyseur à partir du mélange (M).

13. Procédé selon l'une quelconque des revendications 1 à 12, comprenant en outre (iii) la séparation de la sulfone selon la formule (II) à partir du mélange (M), de préférence par précipitation.

14. Procédé selon l'une quelconque des revendications 1 à 13, dans lequel le catalyseur est une poudre pour pulvérisation, facultativement contenue dans un moulage, le moulage comprenant de préférence au moins un liant, de préférence un liant de silice, dans lequel, de préférence, au moins 95 % en poids, plus préférablement au moins 98 % en poids, plus préférablement au moins 99 % en poids de la poudre de pulvérisation est constitué du matériau zéolitique contenant du titane.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RU 2158257 C
- CN 102351757 A
- CN 102351756 A
- WO 2012143281 A
- US 4287366 A
- US 2010120869 A
- CN 102838516 A


### Non-patent literature cited in the description


- *Ullmanns Encyclopedia of Industrial Chemistry,* vol. 13, 447-456
- *CHEMICAL ABSTRACTS,* 3085-42-5